Europäisches Patentamt

(19) European Patent Office

Office européen des brevets

(11) EP 0 533 585 B1

(12) **EUROPEAN PATENT SPECIFICATION**

(45) Date of publication and mention
of the grant of the patent:
**09.12.1998 Bulletin 1998/50**

(51) Int. Cl.$^6$: **A61N 5/06**

(21) Application number: **92402578.6**

(22) Date of filing: **18.09.1992**

(54) **Apparatus for regulating and improving the status of development and survival of living organisms**

Vorrichtung zur Regelung und Verbesserung des Entwicklungs- und Lebenserhaltungszustands lebender Organismen

Appareil pour la régulation et pour améliorer l'état de développementet de survie d'organismes vivants

(84) Designated Contracting States:
**DE FR GB IT NL SE**

(30) Priority: **19.09.1991 CN 91109014**

(43) Date of publication of application:
**24.03.1993 Bulletin 1993/12**

(73) Proprietor: **Zhou, Lin**
**Kunming, Yunnan Province (CN)**

(72) Inventor: **Zhou, Lin**
**Kunming, Yunnan Province (CN)**

(74) Representative:
**Chauchard, Robert et al**
**c/o Cabinet Malémont**
**42, avenue du Président Wilson**
**75116 Paris (FR)**

(56) References cited:
**EP-A- 0 164 836**          **EP-A- 0 291 569**
**DE-A- 3 100 892**          **DE-A- 3 300 517**

• **DATABASE WPIL Week 8851, Derwent Publications Ltd., London, GB; AN 88-361160 & CN-A-85 107 113 (L. ZHOU ET AL.)**
• **DATABASE WPIL Week 9113, Derwent Publications Ltd., London, GB; AN 91-092946 & SU-A-1 581 326 (M. YU GRIGOREV)**

## Description

Field of the Invention

The present invention relates to an apparatus for regulating and improving the status of development and survival of human beings, animals, plants, and micro-organisms, by means of simulated or partially simulated bio-frequency spectrum signals.

Background of the Invention

Modern sciences developed by scientists represented by Isaac Newton have made great contributions to man's understanding of the non-living world. The living world, however, remains to be explored yet. Reductionism holds that the structure and function of large molecules that determine the vital activity is finally reduced to the physical activities of the elementary particles. The discovery of the double spiral of DNA and the study of genetic code well support the reductionism. Vitalism holds that there exist essential differences between living and non-living, and that the vital and independent rules of the vital activity are different from the conventional by systematology, cybernetics, and information theory. A great amount of research work has been done under the guidance of these two kinds of important theories, but a apparatus for effectively regulating and improving the status of development and survival of living organisms has not been found.

The inventor holds that in the non-linear system of life, the nature and status of the isolated individual units due to the decomposition of systems are different from that of the units in an unified system. However, this does not mean the vital activity has nothing to do with the physical and chemical activities. It is known that all things consist of substances, energy, and information. The function of information is particularly important in metabolism, replication, and regulation of living organisms (such as the functions of the genes). The essential difference between living and non-living things is that in living organisms there exists changing information which controls the process and development of life, and represents the normal or abnormal status of the organisms.

Living organisms have unique physical and chemical properties. People know very little about the life information expressed mainly in physical form. According to physical theories, the elementary particles such as molecules, atoms, and electrons are always vibrating and rotating even in their elementary status. Living things are composed of molecules which rotate periodically, and electrons moving surrounding the atoms, while atoms or atom groups are vibrating. These activities form various energy levels and quantizations. The normal state energy of a molecule comprises the following energies.

$$E=Eo + En + Er + Ev + Ee \qquad (1\text{-}1)$$

wherein the energy changes related to the frequency spectrum of radiation of substances are: transitional energy of outer electrons of the nucleus, Ee, rotational energy of the molecule, Er, energy of vibration of the atom, Ev. The corresponding ranges of these three kinds of energy levels are: 1-2oev, 0.05-1ev, and 0.0035-0,05ev, respectively.

Molecules, atoms, and electrons constituting the substances radiate energies in the form of electromagnetic frequency spectrum only during transitions of their energy levels. When the status of a living being changes, the radiant energy and frequency spectrum will change therewith, e.g. cytomembrane potential electrocardiogram or electromyogram will change.

Molecules, atoms, or electrons have their own inherent frequencies. When they are subject to electromagnetic radiation of certain number of vibrations,

$$V=N/C \qquad (1\text{-}2)$$

where V is the number of vibration, N is the frequency, and C is the velocity of light. If the applied electromagnetic frequency is equal to the inherent frequency of the molecules, atoms, or electrons, a condition same as that of resonance in the case of vibration will occur; the molecules, atoms, or electrons will absorb energy and transitions of energy levels can be elicited.

Bohr's quantum condition should be satisfied when molecules, atoms, or electrons transit from one energy level to another through the absorption of energy:

$$Em - En = nVmn \qquad (1\text{-}3)$$

where Em and En are high and low energy levels respectively. Vmn is the number of electromagnetic vibration.

Molecules, atoms, or electrons cannot absorb the electromagnetically radiated energy having the number of vibration Vmn if equation (1-3) is not satisfied. Since the energy levels of molecules, atoms and electrons are different and the number of vibrations are different too, the condition of transitions of energy levels due to the external electromagnetic radiation may be very complicated. The energy absorption will depend not only on the energy of the electromagnetic radiation but also on the wave number of the electromagnetic wave.

Living substances are also composed of molecules, but are more complicated in terms of energy status and structure thereof. It has been found that a living organism is a good natural source of radiation. For example, 45% of the energy of a human body is dissipated in the form of radiant energy. When the temperature of the human body is 37°C, the maximum radiation is deduced from Wien's Displacement Law.

$$max = \frac{2879}{310} = 9.35 \ \mu m$$

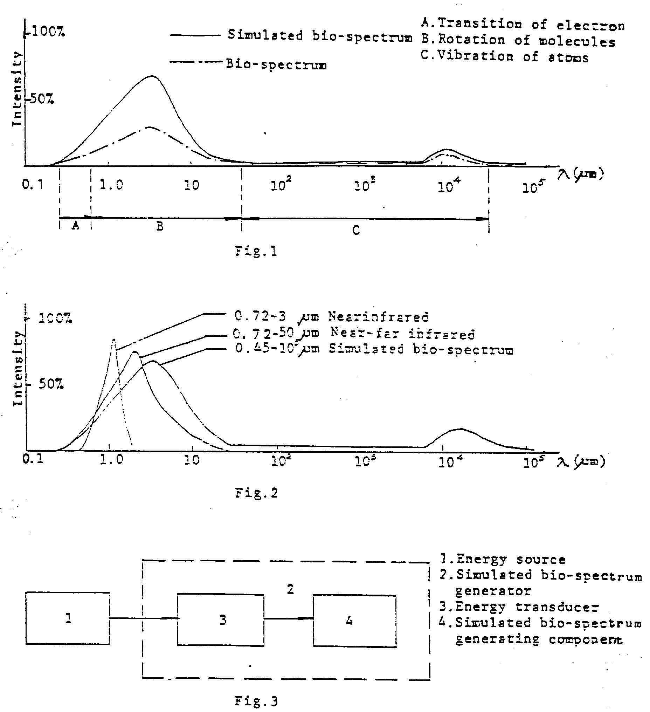

The inventor found through experiments that the band of human frequency spectrum is extremely broad; signals may appear in a band expanding from micrometers ($\mu$m) through millimeters (mm), and including, the near infrared, middle infrared, far infrared, and even millimeter waves. Experiments show that the human body is a comprehensive physical field, including magnetic field, infrared radiations, and weak microwaves (mainly millimeter waves). The inventor holds that using "human spectrum" (Fig. 1, curve A) and according to Kirchhoff's Law, it can be found the absorption spectrum and radiation spectrum are essentially the same. That is to say, the living organisms possess a physical field having certain frequencies and a spectrum which is called by the inventor "bio-frequency spectrum" or "bio-spectrum."

A part of the biospectrum is caused by light radiation of the living organisms, this is the process that chemical energy is effectively transformed into light energy by the organisms. Light radiation by the living organisms may also be called bio-luminescence which is a special case of chemical luminescence. The efficiency of bio-luminescence is very high, i.e. the heat production is very low, so it is commonly called "cold light." Living organisms have to acquire energy through the oxidation of certain easily oxidizable substances to maintain its life. Radicals containing oxygen, for example, OH, may also be utilized to oxidize hard oxidizable hydrocarbons, and biological chemoluminescence can be generated by certain reactions in that process. From microbes to human beings, all living organisms luminesce at a low level. The spectrum is from 180 to 800 nm, the intensity falls into the range of 10 -$10^{-4}$ nv/s.cm, and the quanta produced is only $10^{-14}$ - $10^{-19}$. This kind of luminescence is linked with a number of biological processes such as oxidizing metabolism, detoxication, division and death of cells, photosynthesis, growth regulation, etc. Firefly luminescence is a kind of sex signal containing many detailed structures corresponding to ciphers. The female fly can respond within two seconds after the luminescence of a male fly. Photographs of human luminescence can be taken. A normal human body luminsces mainly from the head; the color of the luminescence is orange-red tinted with a little bluish-green. Red light is emitted from all over the body of a man who is getting angry and forms a thick halo. The inventor thinks that this is due to leakage of the internal energy of the body. When the angry man is irradiated for 15 minutes with the apparatus of the present invention, he will feel relaxed and the anger is relieved. At this time the red light around the body disappears from the photograph, and the status of normal luminescence is resumed.

Cells of the organism can be affected by spectral radiation. Organic molecules generally absorb light with wavelength longer than 200nm, because the energy of the electrons of these molecules is no more than 140 Kcal/mol higher than that of the ground state electrons. Carotinoids absorb light with 450nm wavelength, while purine compounds absorb light with 600nm wavelength light with 254nm wavelength of a flux of $10^8$ photon/cm$^2$ /s has been used to enhance glycolysis in yeast. Light with 80nm wavelength of a flux of $10^5$ - $10^6$ photon/cm$^2$ /s can accelerate the germination of the synchronous yeast cultures.

It can be seen from the above that effects of bio-luminescence, bio-spectrum, and light radiation of various wavelengths on living organisms are obvious. However, conventional studies are still at a low level of understanding said effects and only touch on a very narrow spectral range or a single kind of radiation.

The present invention is capable of regulating and improving growth of the living organism. As an example, when a human body is irradiated with the apparatus, the following regulative effects are produced.

1. Elongation of blood coagulation time and the decrease of blood viscosity at the same time can activate factor 10; the increase of prothrombine activity and the activation of factor 8 at the same time can improve blood circulation, stop bleeding, relieve pain in wounds, eliminate and cure inflammation.

2. Immunological reactions can be changed and phagocyti function is enhanced, marked increase of total number of white cells and lymphocytes in peripheral circulation is achieved, elimination of swelling is better than that in the control group ($P < 0.01$).

3. Certain effects in stimulating the appetite, enhancing the development of the organism, decreasing load on the heart, and the aggregation of blood platelets are achieved.

4. Local hypersensitivity and abnormal feeling caused by damage to the related peripheral nerves are eliminated. Conduction of action potential of the nerves is partly recovered.

5. The apparatus has no direct germicidal effects on isolated bacteria, but effects on the suppurative lesion and dermal ulcer caused by bacterial infection have been demonstrated in rabbits.

6. The present invention can cause the flowability increase of erythrocyte electrophoretic migration rate and morphotropism of red cell membrane, decrease of cell aggregation, which are advantageous to the improvement of rheology of blood and microcirculation.

7. In diabetic rabbit model prepared with tetraoxy pyrimidine, after 1-2 courses of treatment (ten days/course) with the apparatus, the fasting blood-glucose concentration is decreased remarkably and the sugar tolerance curve shifts downwardly.

8. In experiments with a cerebral chemic mice model, the animals in a control group survived for 21.7 hours, while animals treated with the apparatus survived for 41 hours. Symptoms of cerebral ischemia were also milder in the treated group.

9. When patients of disorder in the cervical vertebrane were irradiated with the apparatus, it is found through examination of nail fold microcirculation that the number of capillaries in the nail fold increased, the bore of blood vessels were dilated, and blood flow was accelerated after 10 treatments. The improvement is significant ($P < 0.001$) as compared with conditions before treatment.

10. The apparatus according to the invention is proven to have no deforming effects on mice chromosomes through examination of bone marrow smear.

11. The apparatus according to the invention can enhance growth of microorganisms.

12. The aparatus according to the invention can protect from harmful irradiation.

13. The apparatus according to the inventioin can slow down aging process.

Summarizing the above mentioned experiments and research, the regulative and improving function of simulated bio-spectrum on status of growth of human body can be briefly described as follows:

(1) It makes cells and tissues acquire energy and causes favorable changes in temperature, cell permeability, colloid status, flow rate of body fluid, acidity (pH value), enzyme system and bio-electricity.

(2) It helps in improving and regulating the nervous system through the excitation by the simulated spectral energy. The sequence is: spectral energy --> receptor --> afferent nerve --> nerve center --> efferent nerve --> effector. Regulative reflex arcs are thus formed to maintain biological balance of the human body, and to eliminate diseases and enhance health. Since it functions through reflex of the nervous system, the apparatus can be placed at a distance from the site of the lesion and results in therapeutic effects on the whole body.

(3) The organism can produce certain metabolites and endocrine secretions by the stimulation of simulated human spectral energy. These metabolites and endocrine secretions are conveyed by the body fluid and act on various organs and systems to produce certain physiological regulative reactions and achieve therapeutic effects.

(4) After the nerve is stimulated by the physical field of the simulated bio-spectrum, blood and lymphatic circulations are accelerated, cell vitality and functions of organ systems are enhanced. Thus, the cell and humeral immunities are significantly enhanced.

(5) The signals of the physical field of the simulated bio-spectrum activate regulation of the nervous system, improve body fluid circulation, increase volume of blood vessels, accelerate blood flow, improve blood supply to the heart, enhance peristalsis of stomach and intestine and of body of gland, thus enhance the absorption by the digestive tract and maintains optimal function thereof.

(6) The signals of the physical field of the simulated bio-spectrum stimulate nerves and body fluid to keep physiological balance of the human body through negative feedback and automatically perform the regulative function so that the important physiological indices of human body (such as blood pressure, blood glucose, pH value, blood fat, membrane potentials of organs or cells) are controlled within normal range. Antagonistic symptoms like constipation and diarrohea, bleeding and hemostasis are also improved through the action of negative feedback.

In summary, the present invention can perform at least four distinct functions:

(1) Improving circulation of body fluid;

(2) Regulating nervous system;

(3) Immunological function;

(4) Bio-negative feedback (bilateral regulation).

As human beings are concerned, the above four functions of the invention are sufficient to improve the status of growth, to treat many kinds of diseases and to maintain good health to certain extents. The same functions can also be applied to other species of living beings.

In addition, it has been discovered that the method and apparatus using simulated bio-spectrum can also be applied in biofeedback and the field of oriental traditional medicine.

1. Application in the field of chinese acupuncture and Jing-Luo (main collateral channels).

The apparatus using simulated bio-spectrum is capable of making "Qi" and "blood" flow freely as well as the main and collateral channels unblock (corresponding to the improvements of the regulation of nerves and circulation in western medicine) to achieve the function of regulation and treatment in a manner that the apparatus of the present invention is used to directly irradiate the traditional acupoints, or a large area of the human body. The results are at least similar to those of other oriental traditional methods such as acupuncture and massage.

2. Application in "Qi-Gong" and "Yuga"

Since the function of the present invention is to cause resonating transition of the energy levels of the molecules, atoms, and electrons in the human body, the resonance excited through the acquisition of energy by the cell system reacts on tissues, organs, systems, body fluids, and nerves, and form: a channel for information flow mainly in the form of sensory conduction. If trained with subconsciousness (practicing Yuga or biofeedback), curious sensations of soreness, numbness, fullness, hotness or out crawling can appear at certain parts of the body. These changes can be detected with infrared spectrophotometry. The apparatus of the present invention is applicable in the practice and research of "Qi-Gong" and "Yuga" for information therapy and health maintenance.

The present invention will help in making the exploration of oriental spirit, system, information, and "Qi-Gong" more scientific and standardized.

3. Application in the practice and training of biofeedback.

The principles of the invention can be used in biofeedback practice and training which helps the patients mobilize their intrinsic potentials, substantiate the content of psychotheraphy, treat psychosomatic diseases and train the handicapped extremities.

Up to the present, efforts have been made to control or effect the status of growth and to treat diseases of living beings by means of physical method such as electrotherapy, ultrasound, infrared light, utraviolet light, microwave and laser which treat diseases with different physical factors, but all of these physical means are operating in a single or very narrow range of spectrum, and only resulting in the transition of energy levels in either molecules or atoms or electrons. Although these physical means may produce certain effect in the living beings, the therapeutic effect is still at the lower level.

For example, the ultrasound therapeutic apparatus produces sound wave with frequency over 20,000 Hz to act upon human body; infrared therapeutic apparatus produces infrared light radiation with wavelengths of 0.72 - 25 $\mu$m; ultraviolet apparatus produces ultraviolet rays with wavelengths of 180 - 380 nm; microwave therapeutic apparatus produces ultra-high frequency electromagnetic waves with wavelengths of 1 - 100 mm; while laser treatments use monochromatic laser light to irradiate the human body. All of these methods have deficiencies in therapeutic effects. The reason is that various physical factors do not match with the spectrum of the human body. The natural frequencies of the molecules, atoms, and electrons in the human body.are different from those produced by the various methods, thus cells in the human body are not able to acquire energy and to improve metabolic functions more effectively and the health of the human body is not effectively controlled.

Summary of the Invention

The inventor found through experiments that the status of growth and energy of living beings can be regulated and controlled better only when the energies of the molecules, atoms, and electrons in such living beings are all changed and turned into resonance.

Therefore, the object of the present invention is to provide an apparatus method for regulating and improving the status of growth of the living organisms which affects the growth and survival of living organisms by introducing the signals containing simulated bio-frequency spectrum into the living organisms including human beings, animals, plants, and microbes, to make them match with the inherent spectrum of the organisms.

Another object of the invention is to provide an apparatus for regulating and improving the status of growth of the living organisms which can radiate simulated bio-frequency spectrum similar to the inherent spectrum of the living organisms. Applying such spectrum to certain portions of the living organisms and having it matched with the inherent frequencies of and absorbed by the organisms will result in producing high level regulative reactions and achieving regulation and improvement of the status of growth and survival of the living organisms.

The apparatus for regulating and improving the status of growth of the organisms according to the present invention is defined in claim 1. Apparatuses not according to the invention are merely for illustration.

The apparatus can produce an electromagnetic radiation having very broad spectrum which covers visible light band, near and far infrared band, ultra-far infrared band, submillimeter wave band, and possibly centimeter wave band, i.e., completely covers the frequency wave band of the inherent natural oscillations of the organisms.

The intensities of the electromagnetic radiations are different at different bands of the spectrum, thus the biological effects they elicit are also different. Those eliciting thermal biological effects are in the range of visible light, near, middle, and far infrared. It accounts for more than 90% of the radiated energy. Those eliciting non-thermal biological effects are ultra-far infrared, submillimeter have and millimeter waves which occupy a broad frequency range but a low amount of radiant energy. Measurement with a millimeter wave detector shows that, the radiant energy in the range of millimeter wave can be added directly to the weak radiant energy in the millimeter wave band of the organism for they belong to the same order of magnitude. Non-thermal biological effects are produced by the excitation of the resonance of the external electromagnetic radiation with the elementary particles in the living substances.

The above features make it possible to solve the following two problems:

1. Combining the easily acquirable thermal effect with the non-easily acquirable non-thermal effect brings about good results in the regulation and improvement of growth of the living organisms.

2. The non-thermal biological effect is significant, but difficult to acquire under the low field intensity and low energy conditions of normal temperature and normal pressure. However, the present invention achieves non-thermal biological effects in submillimeter, millimeter, and possibly to the centimeter wave bands with extremely weak radiant energy which is far below the safety standards for electromagnetic waves in many countries and therefore will not cause damage to the living organisms.

These two features are sufficient to produce meaningful complicated biological and physiological processes including neuro-humeral regulation and comprehensive regulative reactions of individual cells and tissue systems which bring the functions of the organism into full play and cure diseases.

BRIEF DESCRIPTION OF THE DRAWINGS

Fig. 1 schematically shows the inherent bio-frequency spectrum of the living organism and the simulated bio-frequency spectrum produced in accordance with the present invention;

Fig. 2 schematically shows the simulated bio-frequency spectrum produced in accordance with the present invention and the spectrum produced with the conventional physical means;

Fig. 3 is the schematic diagram showing the apparatus for regulating and improving status of growth of the organism according to the present invention;

Fig. 4A - 4D are embodiments of the simulated bio-frequency spectrum generator according to the present invention;

Fig. 5A - 5C are schematic diagrams showing regulation and control of the status of growth of the organisms by utilizing the apparatus according to the present invention.

DETAILED DESCRIPTION OF THE PREFERRED EMBODIMENT

In Fig. 1, the broken line denotes the range of distribution of the inherent bio-spectrum, with the wavelengths ranging from 0.2 $\mu$m to 100,000 $\mu$m. The solid line denotes the simulated bio-spectrum generated according to the present invention, wherein 0.45 - 0.72 $\mu$m is the spectral range of eliciting transition of the electrons; 0.72 - 50 $\mu$m is the spectral range of eliciting rotation of molecules; 50 $\mu$m - several cm is the spectral range of eliciting transition of atoms.

Fig. 2 shows one of the current infrared devices (0.72 - 3 $\mu$m) which covers a very short band of the inherent bio-spectrum. The biological effect is mainly thermal. Another one of the current far infrared devices (0.72 - 50 $\mu$m) radiates a wider range of infrared spectra but still a short range in the inherent bio-spectrum. Its main biological effect remains in the thermal effect. The apparatus of this invention which matches the inherent bio-spectrum can generate a very broad range of frequency spectra (0.45 - $10^5$ $\mu$m). The apparatus of this invention can induce more profound biological effects which include thermal and non-thermal effects.

Fig. 3 is the apparatus for regulating and improving the status of the living organism according to the present invention. In Fig. 3, 1 represents the energy source; 3 represents the energy transducer; 4 represents simulated bio-spectrum generating component; energy transducer 3 and generating component 4 constitute the simulated bio-spectrum generator 2. Energy source can be of many forms, such as electrical energy, thermal energy, magnetic energy, solar energy, chemical energy, or biological energy, etc. Electrical energy is preferable because it is easy to acquire. The energy generated by the energy source is transduced into thermal or magnetic energy by energy transducer to provide energy to the simulated bio-spectrum generating component. The simulated bio-spectrum generating component is composed of monomer or compounds of one or more chemical elements in the periodic table. Upon excitation by the energy, the transitions of energy levels of the elements or their compounds are emitted in the form of electromagnetic radiation to form a physical field of simulated bio-spectrum and acts on the organism through radiation. When it matches with the strong absorption band of the organism, a large portion of the radiant energy carried by the electromagnetic wave is absorbed, causing changes of the energies of molecules, atoms, or electrons in the living organism, which then elicits oscillation, enhances bio-oxidation and improves energy of the cells to increase the permeability of cell membrane. With millions and millions of cells reacting in a manner like this, an integrated biological effect is formed, which increases the circulation of body fluid, improves the regulative function of the nervous system, enhances photosynthesis, and finally, engenders the effects in favor of the regulation of growth of the organism. As for the human body, the above four kinds of functions are provided at the same time.

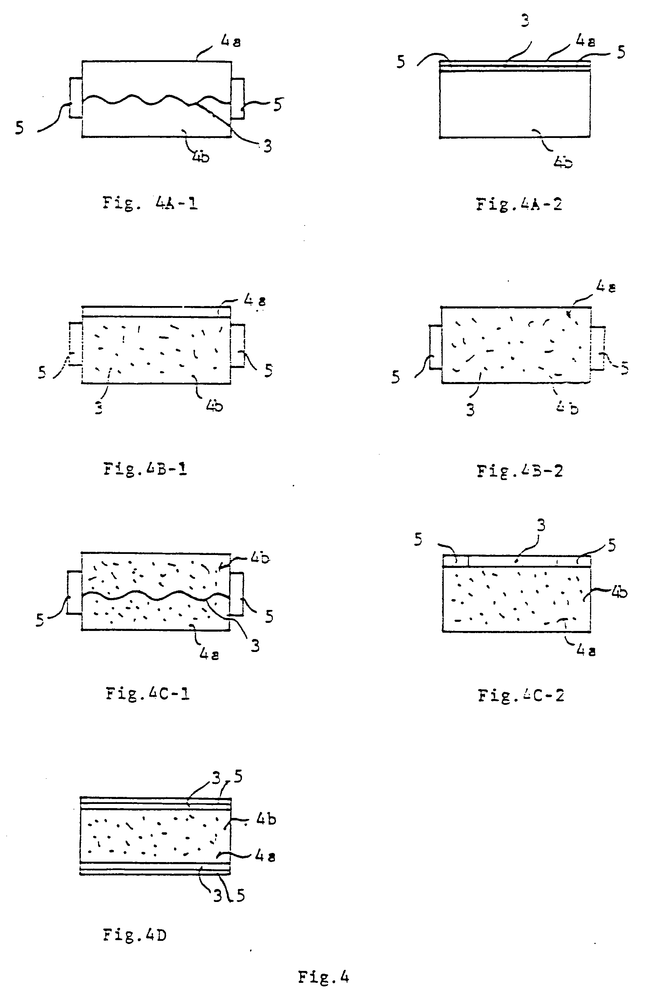

Fig. 4A - 4D show the embodiments of the simulated bio-spectrum generator according to the present invention. In Fig. 4A-1, the simulated bio-spectrum generating component 4 comprises a substrate 4B and an emitting layer 4A disposed on the substrate and composed of borides, nitrides, carbides, sulfides, or fluoride. 5 is the electrode. The proportions of these element and their compounds are determined depending upon the kind of organism to be regulated and its status of growth. This will be described in detail later. Material of the substrate is selected according to the type of energy source which is electrical power source in this embodiment. The substrate can be made from non-metal materials, such as ceramics with low hygroscopic property, high heat resistance, high mechanical strength, high radiance, or heat resistant ( < 150°C) plastics with high radiance or quartz glass, micro cyrstal glass or other kinds of glass with high heat resistance and high strength. It can also be made from electric materials such as carbon rod or resistors which have high electric conductivity and are capable of reaching heat producing temperatures. Various kinds of chemical elements and their compounds are mixed in the right proportion, and then diluted into liquid adhesive, or they may be made into coating material or enamel pulp and coated onto the surface of the substrate to form the emitting layer. Energy transducer 3 can be an electric heating wire or the like which is embedded into the substrate (as shown) or disposed on the ends of the substrate to convert electric energy into thermal energy. The heat generated by the heating wire is used to excite the chemical elements in the emitting layer. The temperature should not be lower than the body temperature of the living organisms

The heating wire 3 in Fig. 4A-1 can be replaced by a layer of conducting membrane 3 (Fig 4A-2) formed on the surface of the substrate by means of metal oxidation technique (high temperature hydrolysis of chlorides to form conducting membrane of metal oxides) to make the simulated bio-spectrum generator more solidified thus increasing the speed and efficiency with which the electric energy is converted into thermal energy (Fig. 4A-1). In Fig 4A-2, the emitting layer 4A is coated on the surface of the conducting membrane.

Fig. 4B-1 is another embodiment of the simulated bio-spectrum generator according to the present invention. In this embodiment, substrate 4b is integrated with the energy transducer 3 in a manner that conducting substance is infiltrated into non-metal material to make it electrically conductive and have satisfactory resistance. The emitting layer 4A is then coated onto substrate to form a simulated bio-spectrum generator. The substrate can also be made from metals. In this case, electric current introduced into the substrate is converted into thermal energy and the substrate acts as the energy transducer at the same time. Then a layer of enamel pulp mingled with one or more chemical elements and their component in the right proportion is coated onto the metal substrate and sintered under high temperature to form a simulated bio-spectrum generator of metal substrate.

In Fig. 4B-2, the chemical elements and their compounds constituting the emitting layer 4A can also be doped into substrate 4B and then sintered under high temperature to form a simulated bio-spectrum generator (Fig. 4B-2) which is even more integral than that of Fig. 4B-1, as shown in Fig. 4B-2.

Fig. 4C-1 is still another embodiment of the simulated bio-spectrum generator according to the present invention. In this embodiment, one or more chemical elements and their compounds are mixed in the right proportion with pot clay and sintered into an integral body so that the substrate per se contains the constituents that generate the simulated bio-spectrum. When energy transducer such as electric heating wire is embedded into the substrate, an integrated simulated bio-spectrum generator is formed.

In Fig. 4C-2, a conducting membrane 3 can be plated onto the surface of the substrate 4B containing the chemical elements of the emitting layer 4A. The conducting membrane 3 replaces the heating wire 3, and makes the simulated bio-spectrum generator a more integral body as shown in Fig. 4C-2.

Fig. 4D is still another embodiment of the simulated bio-spectrum generator according to the present invention. In this embodiment, temperature resisting glass is used as their material of the substrate and one or more chemical elements and their compounds are mixed in the right proportion into the raw glass during sintering. A special glass body containing the constituents that generate the simulated bio-spectrum is formed by sintering. Then a layer of semiconductor membrane is formed on the surface of the glass as the energy transducer by means of the metal oxidation, thus forming a colorless and transparent simulated bio-spectrum generator. In this embodiment, the material of the substrate can also be pot clay containing one or more chemical elements and their compounds so that the substrate per se contains constituents that generate the simulated bio-spectrum. An integrated solid ceramic simulated bio-spectrum generator is formed by disposing a layer of conducting membrane of metal oxides on the surface of such a substrate as an electrically conducting energy transducer.

The substrate can be made from either permanent or electric magnetic materials. In this case, the emitting layer is plated onto the magnetic substrate and a non-thermal simulated bio-spectrum generator made from magnetic material is formed.

The chemical elements to be used in the simulated bio-spectrum generator are selected in accordance with the following principles:

a. The spectrum of radiation of the chemical elements after acquiring energy should be distributed as widely as possible between the micrometer band and the millimeter band. If the radiation is only in the micrometer (infrared) band or only in the millimeter band, the biological effects produced are not good enough. In order to make the produced biological effects favorable to the growth and development of the living organisms, radiant signals should be present all over the range from micrometers to millimeters. Therefore, a broad spectrum of um - mm is a distinct feature of the apparatus of the present invention. Selection and proportion of the chemical elements are indispensable to the realization of the broad frequency spectrum and are based on the fact that the elements must be able to generate a spectrum approximating to the bio-spectrum when excited by energy.

b. The elements should be technically as similar to the chemical constituents of the bio-substances in the living organism as possible. The frequency distribution of the inherent bio-spectrum of the organism is then considered.

The chemical elements the present invention concerns include most of the elements of the 2nd, 3rd, 4th, and 5th periods of the Mendeleev periodical table, and the rare earth elements of the lanthanium and actinium series. Most of these elements are metal elements and are used in the form of oxides, fluorides, nitrides, sulfides, borides, or carbides. The chemical elements used in the apparatus according to the present invention for the purpose of simulating or partially simulating the bio-spectrum of the living organisms are shown in Table A.

Table A

| Chemical elements used in the present invention | | | | | | |
|---|---|---|---|---|---|---|
| Monomer | | Oxides | Carbides | Nitrides | Fluorides | Borides |
| Cobalt | Co | | | | | |
| Copper | Cu | | | | | |

Table A (continued)

| Chemical elements used in the present invention | | | | | | |
|---|---|---|---|---|---|---|
| Monomer | | Oxides | Carbides | Nitrides | Fluorides | Borides |
| Molybodenum | Mo | | | | | |
| Lithium | Li | | | | | |
| Beryllium | Be | BeO | $Be_2C$ | $Be_3N_2$ | | |
| Boron | B | $B_2O_3$ | $B_4C$ | BN | | |
| Magnesium | Mg | MgO | | | $MgF_2$ | |
| Aluminum | Al | $Al_2O_3$ | | | | |
| Silicon | Si | $SiO_2$ | NbC | NbN | | |
| Potassium | K | KO | | | | |
| Calcium | Ca | CaO | | | | |
| Titanium | Ti | $TiO_2$ | TiC | TiN | | $TiB_2$ |
| Vanadium | V | $V_2O_5$ | VC | VN | | $VB_2$ |
| Chromium | Cr | | $Cr_3C_2$ | CrN | | $CrB(Cr_3B_4)$ |
| Manganese | Mn | $MnO_2$ | | | $MnF_2$ | |
| Iron | Fe | $Fe_2O_3$ | | | | |
| Nickel | Ni | NiO | | | | |
| Zinc | Zn | ZnO | | | $ZnF_2$ | |
| Germanium | Ge | GeO | | | | |
| Strontium | Sr | SrO | | | | |
| Zirconium | Zr | $ZrO_2$ | ZrC | ZrN | | $ZrB_2$ |
| Niobium | Nb | | NbC | NbN | | $NbB_2$ |
| Tantalum | Ta | | TaC | TaN | | $TaB_2$ |
| Hafnium | Hf | $HfO_2$ | HfC | HfN | | HfB |
| Selenium | Se | | | | | |
| Thorium | Tn | $TnO_2$ | TnC | TnN | | $TnB_4(TnB_6)$ |
| Tungsten | W | | $WcW_2C$ | | | WB |
| Cerium | Ce | $CeO_2$ | | | | |
| Gold | Au | | | | | |
| Yttrium | Y | $Y_2O_3$ | | | | |

In the course of application of the present invention, one or a plurality of elements and their compounds can be selected from Table A according to the specific living organism to be regulated or the requirements for the simulation or partial simulation of the bio-spectrum.

The mixture ratio of the elements used in the emitting layer of the simulated bio-spectrum generator for different purposes and the applications of the apparatus according to the present invention will now be described by way of examples.

Example 1. Treatment of AIDS

When the apparatus of the present invention is used in the treatment of AIDS, the emitting layer of the simulated bio-spectrum generator contains 16 elements mainly in the form of oxides. The mixture ratio is: chromium oxide 95%, selenium oxide $\geq$ 1%, chromium $\geq$ 0.8%, germanium oxide $\geq$ 0.8%, zinc oxide $\geq$ 0.5%, ferric oxide $\geq$ 0.5%, magnesium

oxide $\geq$ 0.2% copper oxide $\geq$ 0.1%, cobalt oxide $\geq$ 0.1%, manganese oxide $\geq$ 0.1%, molybdenum oxide $\geq$ 0.1%, strontium oxide $\geq$ 0.1%, vanadium oxide $\geq$ 0.1%, aluminum oxide $\geq$ 0.1%, silicon oxide $\geq$ 0.1%, lanthanium $\geq$ 0.1%, magnesium fluoride $\geq$ 0.1%.

In treating AIDS with the apparatus so constructed, single point irradiation, localized multi-point irradiation or general irradiation can be adopted. The principle of treatment is to prevent the onset of the disease and inhibit the antigen (HIVAg) for patients of early infection, to increase the T cells, improve the patient's condition, and to resume immunity for patients of metaphase infection, to control the state of illness, to maintain life so as to alleviate the symptoms and to resume physical strength for patients of late stage.

The process of treatment is as follows:

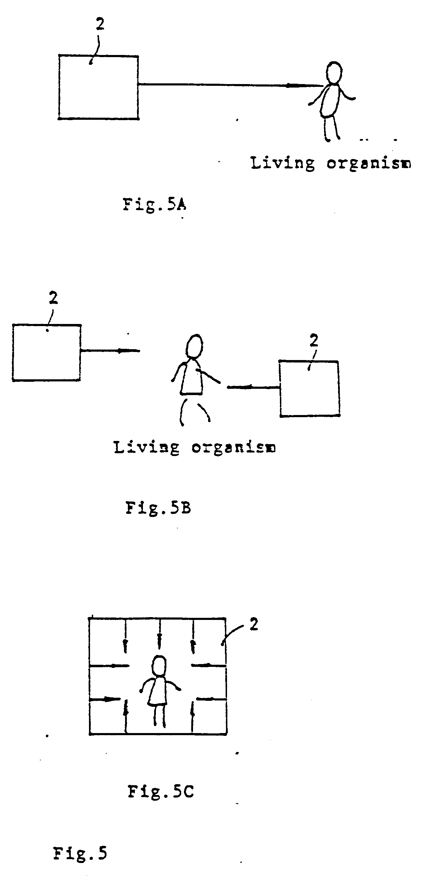

(1) Local single point treatment (Fig. 5A):

Single points on chest, back, abdomen, head, feet, face, cervical vertebrae, and femur are irradiated with the apparatus containing above elements in the emitting layer continuously for at least 5 minutes, 1 - 2 times a day, 30 days a course. Treatment may be conducted course by course without interruption. The therapeutic dose is controlled to the rate with which the patients feel comfortable.

(2) Localized multi-point treatment (Fig 5B):

Simultaneous irradiation to the thymus, spleen, stomach and intestines, vertebrae, femur, head, and feet is required in this case for at least 5 minutes at a time, 1 - 2 times a day, 30 days making one course. There may be no interruption between courses.

(3) General irradiation (Whole body treatment (Fig. 5C)):

The whole body is irradiated for at least 5 minutes a time, 1 - 2 times a day. 30 days make a course and no interruption between courses is strictly required.

(4) For patients in the exacerbation period:

The following process should be followed in addition to those described in (1), (2) and (3):

A. When accompanied with swelling of lymph nodes-irradiation should be directed to bilateral cervical, submaxillary and inguinal lymph nodes symetrically.

B. When accompanied with herpes:--irradiation should be directed to genitalia, anus, and lip.

C. When accompanied with infection and ulcer of the anus (commonly seen in homosexual lovers):--irradiation should be directed to the lesions.

D. When accompanied by pulmonary cysticersus pneumonia (PCP):--irradiation should be directed to the lung, bronchus, and feet.

E. When accompanied with diarrhea:--irradiation should be directed to the abdomen and feet.

F. When accompanied with Kaposi's sarcoma:--irradiation to the lesion and the whole body is required.

G. When accompanied with perleche of buccal cavity and tongue:--irradiation to buccal cavity and tongue, or intrabuccal cavity irradiation is required.

H. When accompanied with epilepsy:--irradiation should be directed to head and feet.

I. When accompanied with fatigue and insomnia:--irradiation should be directed to the abdomen, head, and feet.

J. When accompanied with weakness of memory and headache:--irradiation should be directed to head, abdomen, and feet.

In addition to above four methods of treatment, drugs like AZT, interferon, inhibiting or regulating agents and various nutritive drugs, medical herbs (Chinese medical herbs) may be used together with the irradiation to increase the therapeutic effects or to assist the treatment.

Results of treatment of AIDS:

Twenty-five intermediate or late stage AIDS patients have been treated in accordance with the present invention. Rate of disappearance or improvement of clinical symptom is 70%. The course of treatment is 6 months, 2 times a day. The antigen of AIDS virus (HIVAg) disappeared in 4 cases after treatment. For some of the patients in the early stage of infection of the virus (HIVAg positive), HIVAg was turned to negative after 3 - 6 months treatment.

Typical case 1, a male of 42 years from New York. Before treatment: thin and fatigued with swollen lymph nodes, T4: 350, HIVAg: 164. After two months treatment: fatigue disappeared, swelling of lymph nodes subsided, subjective feeling was well. T4: 460, HIVAg negative.

Typical case 2, a male from New York. Before treatment: fatigue, thin and extremely exhausted, accompanied with cysticercus pneumonia, anal herpes and Kaposi's sarcoma, T4: 1 (normal value is 404--1700). After treatment: T4: 1, HIVAg not detected, fatigue relieved, cysticercus pneumonia and anal herpes disappeared, Kaposi's sarcoma of the nose subsided, body weight increased by 15 pounds.

Example 2. Treatment of Hepatitis

When used in the treatment of hepatitis, the emitting layer of the simulated bio-spectrum generator of the apparatus according to the present invention should contain:

| | |
|---|---|
| chromium oxide 95% | ferric oxide $\geq 0.5\%$ |
| chromium $\geq 0.8\%$ | zinc oxide $\geq 0.4\%$ |
| copper oxide $\geq 0.1\%$ | cobalt oxide $\geq 0.1\%$ |
| manganese oxide $\geq 0.1\%$ | molybdenum oxide $\geq 0.1\%$ |
| selenium oxide $\geq 0.7\%$ | strontium oxide $\geq 0.1\%$ |
| vanadium oxide $\geq 0.1\%$ | aluminum oxide $\geq 0.1\%$ |
| magnesium oxide $\geq 0.1\%$ | silicon oxide $\geq 0.1\%$ |
| germanium oxide $\geq 0.6\%$ | lanthanium $\geq 0.1\%$ |
| Boric oxide $\geq 0.1\%$ | magnesium fluoride $\geq 0.1\%$ |

When the apparatus so constructed is used to treat various types of hepatitis and cirrhosis of liver, the irradiation is directed to liver region, stomach region, vertebrae, feet, and hands individually or collectively. Whole body irradiation may also be used in this case.

The course of the treatment is 30 days with 1 - 2 times a day and 10 - 60 minutes a time, 1 - 7 courses are needed.

Dosage: The simulated bio-spectrum generator of the apparatus is placed 20 - 60 cm from the human body. It is preferable that the patient feels warm and comfortable.

Drugs like immunity regulating agents or nutritives can be used during the treatment.

Effects of treatment:

Fifty cases of type B hepatitis were treated with another 50 cases as control group. After one month of treatment, total protein (TP) increased apparently ($P < 0.05$), while albumin (A) increased significantly ($P < 0.01$). Rate of relief or disappearance of clinical symptom is 85%, serological and liver function parameters are improved, positive turning rate of HBSAg surface antigen is higher than 20%. In addition, 11 cases of cirrhosis of liver accompanied with swelling of spleen were treated. Albumin (increased apparently ($P < 0.05$). Type B ultra-sound follow-up examination shows spleen has shrinked continuously; in four cases, spleen has shrinked to within normal range.

Mechanism of treatmment of hepatitis:

Type B hepatitis is caused by the continuous duplication of HBV, which results in chronic immunological damage to liver tissue. At early stage, the pathologic change in liver tissue is mainly degeneration of cells and inflammatory infiltration. As the change proceeds further, focal necrosis of liver cells, proliferation of fibrous tissues and damage to lobular plates and bridge formation may occur. When the lesions develop continuously, cell necrosis and accretion will be repeated, pseudo-lobulars will be formed continuously and portal cirrhosis of liver will ensue. In the treatment of the hepatitis using the apparatus of the present invention, the bio-spectrum acts directly on the elementary mass particles (molecules, atoms, electrons) in the liver cells to cause transitions and acquisition of energy by the liver cells. Due to the biological effects produced in the cells, organs and tissue systems by the bio-spectrum of the present invention, it is possible to stop the development of diseases effectively and in a timely fashion, to enhance the biochemical metabolism and the generation of normal cells, and to improve circulation in the liver tissue and parameters like serum albumin, and finally to achieve the goal of treatment.

Example 3. Treatment of Tumors

The apparatus of the present invention can be used for the treatment of many kinds of tumors. In the apparatus for the treatment of tumors, the emitting layer of the simulated bio-spectrum generator should include the following constituents:

| | |
|---|---|
| chromium oxide 93% | ferric oxide $\geq$ 0.5% |
| chromium $\geq$ 0.8% | zinc oxide $\geq$ 0.5% |
| copper oxide $\geq$ 0.1% | cobalt oxide $\geq$ 0.1% |
| manganese oxide $\geq$ 0.1% | molybdenum oxide $\geq$ 0.1% |
| selenium oxide $\geq$ 1% | strontium oxide $\geq$ 0.1% |
| vanadium oxide $\geq$ 0.1% | aluminum oxide $\geq$ 0.1% |
| magnesium oxide $\geq$ 0.1% | silicon oxide $\geq$ 0.1% |
| germanium fluoride $\geq$ 0.1% | lanthanium $\geq$ 0.1% |
| magnesium fluoride $\geq$ 0.1%. | |

Elements like lithium, potassium, and titanium may also be added, but the content of each of the should not exceed 0.2%.

In order to accurately confirm the therapeutic effects of the apparatus of the present invention containing the above constituents, experiment was made using the cross-bred kunming mice provided by the animal center of the Chinese Academy of Medical Science as test subjects.

Cells of ascitic type liver cancer $H_{22}$ were inoculated, ascitis was accumulated 48 hours later. The mice were then randomly divided into four groups and irradiated with the apparatus of the present invention containing the above constituents at a distance of 20 cm, one time (20 minutes) a day. the dose was 5 - 30 mW/cm$^2$. The control group was injected with Pin Yang Mei Su, an anti-cancer drug produced by the Hobei Drug manufactory, Tianjin, China, intra-peritoneally 0.1 mg every other day.

Analysis of ascitic fluids which were randomly withdrawn 10 days later shows that in the treated mice, infiltration around tumor cell was apparent, the number of lymphocytes increased from 22 to 41 (both B cells and T cells of the lymphocytes have anti-cancer action) and number of cancer cells were apparently less than those in the control group. Apparent changes in DNA content in cancer cells in the mice treated with the bio-spectrum were proved by morphmetering of the nucleus by image analysis system, DNA multi-body determination, nucleolus surface determination and determinations of total number, and size of granulus.

Example 4. Radiation Protection

Damage caused by radiation can be protected and reduced by using the apparatus of the present invention. The emitting layer of the simulated bio-spectrum generator for this purpose should contain the following:

| | |
|---|---|
| chromium oxide 94% | ferric oxide $\geq$ 0.5% |
| chromium $\geq$ 0.8% | zinc oxide $\geq$ 0.7% |
| copper oxide $\geq$ 0.2% | cobalt oxide $\geq$ 0.2% |
| manganese oxide $\geq$ 0.3% | molybdenum oxide $\geq$ 0.1% |
| selenium oxide $\geq$ 0.9% | strontium oxide $\geq$ 0.1% |
| vanadium oxide $\geq$ 0.1% | aluminum oxide $\geq$ 0.1% |
| magnesium oxide $\geq$ 0.1% | silicon oxide $\geq$ 0.1% |
| germanium oxide $\geq$ 0.8% | lanthanium $\geq$ 0.1% |
| $CaCO_3$ $\geq$ 0.1% | |

Experimentation was made on protection of animals from radiation injury using the apparatus described in this example.

Kunming mice, guinea pigs (serum) and sheep (whole blood) were used in the experiment. The animals were irradiated externally with -ray of $^{60}$Co and internally with $^3$H-water -ray, then treated with the apparatus of the present invention (preventive treatment before the irradiation serves the same function) two times a day, 30 minutes each time, 30 days a course. The apparatus was placed 25 cm from the animals and the dose was controlled to 5 - 30 mW/cm$^2$. The results are shown in Table 1:

Table 1

| Results of experiment with the apparatus of the present invention | | | | |
|---|---|---|---|---|
| Series no. | Parameters | Reaction to Irradiation | Reaction to Irradiation plus bio-spectrum (before or after irradiation) | Beneficial Effects |
| 1 | Blocks walked in open field | 100 | 130 | Increase of nerve excitability |
| 2 | Times of probing on a punched | 100 | 110 | Increase of ability of exploration |
| 3 | Avoidance of electric shock on platform | 100 | 110 | Increase of learning memory |
| 4 | Number of deformed bone marrow cells | 100 | 74 | Reduction of radiation injury |
| 5 | Slit in single chromosome | 100 | 21 | Reduction radiation injury |
| 6 | Survival of mice | 100 | 120 | Increase of survival rate |
| 7 | Number of PFC/$10^5$ spleen cells | 100 | 330 | Increase of immunologic function |
| 8 | Spleen index of mice | 100 | 130 | Increase of immunologic function |
| 9 | Rate of micronucleus of lymphocytes | 100 | 76 | Reduction of radiation injury |

The nine biomedical indexes in Table 1 shows that the simulated bio-spectrum has anti-radiation effects or can prevent or reduce the damage from radiation.

Example 5. Delay of Natural Degeneration of the Living Organisms

The simulated bio-spectrum generator of the present invention can be used to affect the tendency of natural degeneration of the living organisms when the following constituents are added:

chromium oxide 94%  ferric oxide $\geq$ 0.5%
chromium $\geq$ 0.8%  zinc oxide $\geq$ 0.8%
copper oxide $\geq$ 0.2%  cobalt oxide $\geq$ 0.2%
manganese oxide $\geq$ 0.3%  molybdenum oxide $\geq$ 0.1%
selenium oxide $\geq$ 0.9%  strontium oxide $\geq$ 0.1%
vanadium oxide $\geq$ 0.1%  aluminum oxide $\geq$ 0.1%
magnesium oxide $\geq$ 0.1%  silicon oxide $\geq$ 0.1%
germanium oxide $\geq$ 0.8%  lanthanium $\geq$ 0.1%
KI $\geq$ 0.1%  $BO_2 \geq$ 0.1%
$CaCO_3 \geq$ 0.1%  $MgF_2 \geq$ 0.1%

To verify the effects of the present invention in this regard mice were irradiated with the said apparatus continuously for 48 days, 2 times a day, 35 minutes a time, at a dose of 5 -30 mW/cm$^2$ and observation was kept on the natural degeneration of the fur and the naturally swollen manubrium sterni. The results are shown in Tables 2 and 3.

Table 2

| The effects of simulated bio-spectrum on the natural degeneration of fur in mice | | | | | | |
|---|---|---|---|---|---|---|
| Group | Number of | Tail tip | Middle segment | Tail tip | Middle segment | Total % |
| control simulated | 57 | 7 | 1 | 4 | 12 | 21 |

Table 2 (continued)

| The effects of simulated bio-spectrum on the natural degeneration of fur in mice | | | | | | |
|---|---|---|---|---|---|---|
| Group | Number of | Tail tip | Middle segment | Tail tip | Middle segment | Total % |
| bio-spectrum | 53 | 5 | 0 | 0 | 5 | 9.4 |

The white turning rate of the fur on the tail was 21% in the control group, while only 9.4% in the bio-spectrum treated group. Hence there is a tendency of delaying the natural degeneration.

Table 3

| Effects of simulated bio-spectrum on the swelling of manubrium sterni | | | |
|---|---|---|---|
| Group | Number of mice | Natural swelling of manubrium sterni | P value |
| control | 57 | 10 (17.5%) | P < 0.001 |
| simulated bio-spectrum | 53 | 1 (1.9%) | |

In Table 3, the percentage of swelling of manubrium sterni in the control group is 17.5%, while that of the bio-spectrum treated group is only 1.9%. P < 0.001 as verified with the x test.

Example 6. Treatment of Epilepsy

When the apparatus of the present invention is used for the treatment of epilepsy, the following constituents should be added into the simulated bio-spectrum generator:

chromium oxide 94%          ferric oxide $\geq$ 0.5%
chromium $\geq$ 0.8%          zinc oxide $\geq$ 0.5%
copper oxide $\geq$ 0.1%          cobalt oxide $\geq$ 0.1%
manganese oxide $\geq$ 0.1%          molybdenum oxide $\geq$ 0.1%
selenium oxide $\geq$ 0.9%          strontium oxide $\geq$ 0.1%
vanadium oxide $\geq$ 0.1%          aluminum oxide $\geq$ 0.1%
magnesium oxide $\geq$ 0.1%          silicon oxide $\geq$ 0.1%
germanium oxide $\geq$ 0.8%          lanthanium oxide $\geq$ 0.1%

and a small amount of metal elements such as lithium, potassium, and sodium, their total amount should not exceed 0.5%.

To treat epilepsy, head, neck, feet, and hands are irradiated two times a day, 45 minutes per time with 12 days making a course. The sites to be treated must be naked and the dose is controlled to that with which the patients feel comfortable.

Mechanism of treatment and therapeutic effects:

The brain is the highest nervous center. The metabolism of brain tissue depends strictly on the continuous supply of circulated blood to the brain. Damage to the hypothalamus by whatever reason can cause attack of autonomic epilepsy. Epilepsy is also closely related to changes in the cerebral vessels, cerebral blood flow (CBF), and oxygen demand. Since the simulated bio-spectrum of um to mm is acted on the brain, mainly on the hypothalamus cells, to improve its conditions, the improvement of the blood circulation in the brain and regulation of the autonomic nerves, sympathetic nerves and brain can prevent the formation of abnormal discharges of the epileptic focus. Clinical use of the apparatus for the treatment of 45 cases together with 30 cases treated with conventional drugs as control group shows that the effective rate of a 40 day course treatment is > 90%, P < 0.05 as compared with that of the control group. Abnormality rate of electro-encephalogram (EEG) of the patients is reduced by 55% after treatment, while the normal rate increased by 1.4 times. The abnormality rate of reoencephalogram (REG) of the patients is reduced by 45.72%, while the normal rate increased by 1.9 times after treatment. Recurrence occured in all of the control cases within 7 - 30 days, while there had been no recurrence in the cases treated with the apparatus within six months. In addition, anxiety, nervousness, and depression were eliminated.

Example 7. Quick Healing of Wounds and Burns

The apparatus of the present invention is applicable in accelerating the healing of various wounds and burns by adding the following constituents into the simulated bio-spectrum generator:

| | |
|---|---|
| chromium oxide 95% | ferric oxide $\geq$ 0.5% |
| chromium $\geq$ 0.8% | zinc oxide $\geq$ 0.5% |
| copper oxide $\geq$ 0.1% | cobalt oxide $\geq$ 0.1% |
| manganese oxide $\geq$ 0.1% | molybdenum oxide $\geq$ 0.1% |
| selenium oxide $\geq$ 0.9% | strontium oxide $\geq$ 0.1% |
| vanadium oxide $\geq$ 0.1% | aluminum oxide $\geq$ 0.1% |
| magnesium oxide $\geq$ 0.2% | silicon oxide $\geq$ 0.1% |
| germanium oxide $\geq$ 0.8% | lanthanium $\geq$ 0.1% |

To treat various wounds and burns, the apparatus is used 1 -2 times a day, 35 minutes a time. Irradiation from the apparatus is directed to sites of treatment, i.e., opening or surface of the wounds which should be naked and cleaned. Therapeutic effects are remarkable. In the treatment of burns, the pain is rapidly relieved; a membrane is formed, sealing the surface of burn and the restoration stage starts immediately thereafter so that dangerous acute infections are prevented. Since it can improve the microcirculation, metabolism, and regeneration of blood vessels, tissues are enhanced very significantly. As exemplified by second degree burns, the wounds of the patients in the treated group were healed in four days on the average (treated with the apparatus one time a day) with the wound surface being exposed completely during the treatment. While the control cases were treated with dressings change, injections, and medications and healed in 15 days on the average accompanied with pain. At early stages of the wound, the apparatus according to the invention can relieve the patients from pain rapidly, reduce exudation from the wound surface, and facilitate the generation of fiber cells, T cells, and white cells to strengthen the ability of healing. Since the circulation of body fluid is enhanced, the products of inflammation are well absorbed, and the scars formed are slight, healing is apparently accelerated, which enables the patients to pass through the course with little anxiety or pain.

The most distinct changes of healing during treatment of the wounds with the apparatus according to the invention take place on the 2nd - 4th day when the following phenomena of healing appear (incisional wound observed under microscope):

(1) W B C in superficial layer of skin changes from "distinct" to "disappeared";

(2) lymphocytes in dermis layer change from "many" to "little";

(3) fibroblasts proliferate as the healing begins, then reduce in number (representing well healed);

(4) tissue edema disappear (representing healed).

All of these phenomena appear 1 - 3 days earlier than in the control group.

Results of clinical experiment on abdominal incisional wound show that the stitches were removed after seven days with the conventional method of treatment while stitches could be removed only four days (80 - 96 hours) after the operation when the apparatus is used according to the invention, and the standard of class A healing was achieved. Both pain and infection were also apparently controlled and administration of antibiotics were not needed. Body temperature returned to normal in 80 hours. Local tenderness of wound was reduced.

Example 8. Treatment and Prevention of Common Cold

Common cold can be treated with the apparatus described in example 7 in a manner that head, nose, buccal cavity, throat, neck, feet, and knees are irradiated 1 - 2 times a day, 30 - 120 minutes a time for a course (1 - 7 days). When used for the prevention of common cold, irradiation for three months continuously (1 - 2 times a day) is needed at the beginning. Then it can be reduced to three times a week.

Therapeutic effects: At the early stage (on the day of appearance of the symptom, or Catarrh stage), the effective rate is > 70% after only one time of treatment. Body temperature can be reduced to normal after two hours treatment in cases with ordinary fever. For cold at other stages, symptoms can be relieved, time of convalescence shortened and complications prevented.

Example 9. Treatment of Herpes

Simple herpes or herpes zoster can be treated with the apparatus described in example 7, 1 - 2 times a day, 40 minutes a time, for at least a course (5 days). The sites of treatments are now the lesions of the herpes, lymph node, feet, and spleen.

Therapeutic effects are remarkable for painful symptoms were eliminated or relieved in 1 - 3 days, blisters subsided distinctly, rate of relapse reduced after 1 - 4 courses of treatment. An effective rate higher than 90% was confirmed in 50 cases, $P < 0.01$ as compared to the control group.

Example 10. Treatment of Angiocardiopathy and Cerebrovascular Disease

The apparatus can be used in the treatment of angiocardiopathy and cerebrovascular disease when the following constituents are added into the simulated bio-spectrum generator:

| | |
|---|---|
| chromium oxide 92% | ferric oxide $\geq 0.5\%$ |
| chromium $\geq 0.8\%$ | zinc oxide $\geq 0.7\%$ |
| copper oxide $\geq 0.1\%$ | cobalt oxide $\geq 0.1\%$ |
| manganese oxide $\geq 0.2\%$ | molybdenum oxide $\geq 1\%$ |
| selenium oxide $\geq 1\%$ | strontium oxide $\geq 0.1\%$ |
| vanadium oxide $\geq 0.1\%$ | silicon oxide $\geq 0.1\%$ |
| germanium oxide $\geq 0.1\%$ | lanthanium $\geq 0.1\%$ |
| KI $\geq 0.1\%$ | |

Suitable amount (no more than 0.2%) of elements like titanium or boron are added.

In treating such diseases, irradiation is directed to precordial region, feet, hands, vertebra, neck and head 1 - 2 times a day, 20 - 60 minutes a time, for at least one course (12 days).

Therapeutic effects: Since volume of blood vessels and stroke volume of the heart are increased due to the irradiation by the apparatus, blood and oxygen supply to the heart and brain are significantly improved, which in turn improves function of the heart and brain. Electrocardiogram and electroencephalogram are also improved. So the apparatus is applicable in treating many kinds of diseases of cardiac or cerebral tissues or blood vessels. These effects are verified by animal experiments and clinical observations. It is very effective in eliminating premature beats or inverted T wave in virus myoearditis and changes of S-T segment due to coronary blood supply insufficiency, and also capable of regulating and improving cystolic conditions of the heart.

Clinical trial of the treatment of hypertension demonstrated that after 10 days treatment with the apparatus of the present invention by irradiating the underside of the foot, precordial region, hands, arms, and cervical vertebra, systolic blood pressure dropped by 7.1% (11.4 mm Hg, $P < 0.002$), diastolic blood pressure (DBP) dropped by 7.9% (7.9 mm Hg, $P < 0.001$) in the treated group. The total effective rate is 54.3% (N=25). If the course of treatment is longer than 10 days, the effective rate will increase obviously and the rate of lowering of blood pressure will be higher than 80%.

Clinical trial of the treatment of coronary heart disease demonstrated that the effective rate of treatment of coronary heart disease with the apparatus is higher than 85%, improvement in myocardial iscemia and cardiac function is apparent and statistically significant ($P < 0.01$).

The criteria of improvement of myocardial ischemia are relief of angina pectroalis, recovery of ST-T changes, elevation of depressed ST more than 1.0 mm.

The criteria of improvement of cardiac function are as follows: the cardiac function is lowered by one grade and reaches grade 0 or 1; the symptoms are relieved.

Example 11. Treatment of Rheumatoid Arthritis and Other Diseases of Joints or Soft Tissues

Rheumatoid can be treated with the apparatus described in example 7 in a manner that the diseased joints, spleen, femur, and feet are irradiated 1 - 2 times a day, 30 - 60 minutes a time, for 1 - 6 courses (20 days make a course).

The treatment can be assisted by externally administered medications such as levamisole applied to the skin or the diseased joint to reinforce the therapeutic effect.

The effective rate is higher than 60%. Pain is relieved or eliminated obviously.

106 cases of lumbago (osteoarthritis, damage of the soft tissue) were treated with the present apparatus. The index of lumbago was reduced remarkably ($P < 0.001$), while that of the control group treated with conventional medication and acupuncture showed no significant reduction ($P > 0.051$).

Example 12. Human Health Care

The apparatus of the present invention can be used in health care for human body when bio-spectrum in micrometer wavelength range (visible light to 25 μm) is simulated. The simulated bio-spectrum generator should contain the following constituents:

| | |
|---|---|
| selenium oxide ≥ 5% | germanium oxide ≥ 5% |
| silicon oxide ≥ 50% | thorium oxide ≥ 4% |
| cerium oxide ≥ 5% | zirconium oxide ≥ 15% |
| boron nitride oxide ≥ 10% | titanium carbide ≥ 3% |
| tungsten carbide ≥ 3% | |

Besides, platinum 0.01% is added.

When the apparatus described in this example is used in health care for human body, it is capable of enhancing body fluid circulation, regulating the nervous system, and strengthening psychosomatic quality. Thus the incidence of commonly encountered diseases like common cold are significantly reduced, digestive functions of stomach and intestine, sleeping, blood pressure, heart rate, and cardiac and cerebral functions are improved after three months treatment with the above apparatus. It is particularly effective in the early stage of diseases and discomfort caused by climates, environments, psychological factors, and foods, and capable of helping the human body to regulate and eliminate the internal changes in certain degrees, so that balance of the internal physical and chemical conditions and health of the human body are maintained.

Example 13. Elimination of Fatigue and Enhancement of Recovering of Athletes.

The apparatus of the present invention can be used to eliminate fatigue, assist recovery, and increase physical ability and competition power of the professional athlete. The emitting layer of the simulated bio-spectrum generator should contain the following constituents:

| | |
|---|---|
| aluminum oxide ≥ 85% | ferric oxide ≥ 3% |
| cobalt oxide ≥ 2% | titanium oxide ≥ 3% |
| silicon oxide ≥ 3% | hafnium oxide ≥ 2% |

Besides, platinum 0.01% is added.

In eliminating fatigue in athletes and assisting recovery, the apparatus is used to irradiate undersides of the feet, abdomen, lumbar region, kidney region, or the whole body 1 - 2 times a day, 30 minutes a time. It is preferable to use it after intensive exercise for elimination of fatigue, or to use it one hour before competition for regulating psychosomatic condition effectively.

Therapeutic effects: a 30-day trial was made on a group of 46 young or juvenile athletes (24 male and 22 female) engaged in items of endurance with daily irradiation of 30 minutes. The assigned exercise was running along a road with a slope of 150° and at a speed of 10 km/hr. for six minutes. The results show that blood lactic acid level was loweer than that before irradiation, flicker fusion threshold increased, reaction time shortened, gripping force and strength of the lumbar and back muscles increased significantly.

Conclusions: the biological effects produced by the apparatus of the present invention function through cells receiving the effects and then casting influence on the neuro-endocrine system, especially the sympatho-adrenal system. It is capable of increasing aerobic capacity, improving the psychosomatic condition, increasing appetite and improving dream state, and therefore effective in assisting the recovery from fatigue caused by exercise, and in eliminating the fatigue of central origin.

Example 14. Treatment of Disease of the Five Organs

The apparatus of the present invention can be used to treat diseases of the ear, nose, throat, eye, and buccal cavity when the following constituents arc added into the simulated bio-spectrum generator:

| | |
|---|---|
| yttrium oxide ≥ 5% | ReCaMnFeOx ≥ 90% |
| silicon oxide ≥ 5% | |

In treating diseases of the five organs, the treatment should be conducted 1 - 2 times a day, 20 - 40 minutes a time, with 12 days making a course. Significant effects can be obtained after 1 - 4 courses. The sites of treatment are mainly the sites of the lesions.

Examples of application:

Ear:            otitis media, impaired hearing;
Nose:           various kinds of rhinitis;
Throat:         inflammation of pharynx or larynx;
Buccal cavity:  ulcers in buccal cavity;
Eyes:           conjunctivitis, juvenile myopia

Therapeutic effects: the effective rate of treatment of diseases of the five organs is ≥ 80% on the average. Impairment of circulation and nerves are eliminated timely and surgical operation can be avoided. Effective rate of treatment of juvenile pseudo-myopia is ≥ 85%.

Example 15. Treatment of Diseases of the Kidney

The apparatus of the present invention can be used for the treatment of diseases of the kidney when the following constituents are added into the simulated bio-spectrum generator:

chromium oxide ≥ 96%      chromium ≥ 1%
zinc oxide ≥ 1%           silicon oxide ≥ 1%

The treatment should be conducted 1 - 3 times a day, 20 - 30 minutes a time with 30 days making a course. The sites of treatment are: kidney region, abdomen, undersides of the feet, lumbar region, leg, or the whole body.

Therapeutic effects: in seven cases of chronic renal failure (CRF), mean BUN decreased by 32.5 mg/dl, mean urine volume increased by 255 ml/d, hemoglobin and scr improved after treatment (P < 0.05). Therapeutic effects were obtained without dialysis. The present apparatus is capable of improving the filtering and eliminating function of the renal glomeruli, and of eliminating the accumulation of metabolites of protein. Thus, diseases of the kidney are cured effectively. General post-treatment weakness and symptoms of the digestive tract are improved significantly. SOD and CGRP are also improved.

Various kinds of diseases of the kidney, including renal hypertension, can be treated with this apparatus. It is also effective in preventing the repelling reaction after transplantation of kidney.

Example 16. Treatment of Hemorrhoid

The apparatus of the present invention can be used for the treatment of hemorrhoid when the following constituents are added into the simulated bio-spectrum generator:

titanium oxide ≥ 90%      zirconium oxide ≥ 5%
silicon oxide ≥ 1%        ferric oxide ≥ 1.5%
zinc oxide ≥ 1%           copper oxide ≥ 1%
beryllium oxide ≥ 0.5%

In treating hemorrhoid, lesions at the anus are irradiated 1 - 2 times a day, 20 - 40 minutes a time (12 times make a course). Apparent effects were obtained in 70% of cases after one course treatment. Undersides of feet and abdomen can be irradiated for prevention of exacerbations.

Therapeutic effects: In 64 cases of thrombosed hemorrhoid, 60 cases (93.75%) were cured substantially after one course of treatment. Inflammation and swelling subsided and pain was relieved after 2 - 3 times of treatment. No medication is needed during the treatment. The functions of improving blood circulation and regulating the nervous system performed by the present invention act on the lesion simultaneously, which accelerates the exchange of substances between bloom and tissues, increases function of the reticulo-endothelial system, enhances phagocytic power of WBC, reduces muscle tone, loosens sphincter ani and reduces tract pressure, and therefore causes the swelling to subside in a timely fashion, leading to the thrombosed hemorrhoid being cured effectively.

Example 17. Treatment of Menorrhalgia and Gynecologic Disease

The apparatus of the present invention can be used to treat menorrhalgia and gynecologic diseases when the following constituents are added into the simulated bio-spectrum generator:

silicon carbide monomer (high purity) 90%

chromium 2%                                                    zinc oxide 1%
ferric oxide 1%.

When used for the treatment of menorrhalgia and gynecologic diseases (inflammation and sterility), the apparatus of the present example is operated to irradiate the sites of treatment 1 - 2 times a day, 20 - 40 minutes a time. A course for the treatment of menorrhalgia contains seven times of treatment while a course for the treatment of other gynecologic diseases contains 30 times of treatment.

The sites of treatment are sites of pain and inflammation, abdomen, lumbar region, perinial region, and undersides of the feet.

Therapeutic effects: In 79 cases of menorrhalgia, 65 cases (82.3%) were cured and obvious effects were obtained in 10 cases (12.7%).

Among 52 cases of inflammation in the pelvic cavity and its appendix tested, 14 were cured, 36 improved, and 2 ineffective. The effective rate is higher than 90%. The period of treatment needed was 20 days on the average.

15 cases of erosion of cervix uteri were tested, 5 cured, 9 improved, and no effect in 1 case. The period of treatment was 10 days on the average.

It is also effective for treatment of sterility, but the observation is not completed yet.

Example 18. Treatment of Respiratory Diseases

The present invention can be used for treatment of respiratory diseases when the following constituents are added into the simulated bio-spectrum generator:

ferric oxide $\geq$ 20%              zirconium oxide $\geq$ 75%
silicon oxide $\geq$ 2%              zinc oxide $\geq$ 1%
niobium oxide $\geq$ 2%

When used for the treatment of respiratory disease, the apparatus should act on the patients 1 - 3 times a day, 20 - 40 minutes a time (30 days make a course).

Sites of treatment: bronchus, chest, back, undersides of the feet, preferably the chest and back at the same time.

Therapeutic effects: good anti-asthmatic effects can be obtained in treating bronchial asthma. When chronic asthmatic bronchitis cases accompanied with emphysema are treated for three courses the asthmatic symptoms can be relieved. Preventive results can be achieved for respiratory bronchial asthma.

Cases of acute bronchitis and pneumonia are cured after 7.7 days (average) of treatment, while the control group cured after 9.05 days. The difference is significant ($P < 0.05$).

The mechanism of suppressing the asthma and eliminating inflammation is to increase non-specific immunologic mechanism and anti-allergic action, regulate and balance autonomic nervous functions, reduce excitability of sympathetic nerve, stop cough and sputum, suppress asthma. The apparatus has an anti-infectious function, and is capable of suppressing asthma immediately. It is also effective for emphysema or cor pulmonale.

Effectiveness in treatment of respiratory diseases can be enhanced by accompaniment of medications.

Example 19. Treatment of Diseases of Stomach and Intestine

The apparatus of example 18 can also be used for the treatment of diseases of stomach and intestine. The treatment should be conducted 1 - 3 times a day, 20 - 40 minutes a time. The course for diseases of intestine is seven days.

Sites of treatment are gastric and intestinal regions, knee joint, undersides of the feet.

Therapeutic effects: gastric ulcer and gastritis treated for one course, effective rate > 90%. Chronic gastritis treated for one course, effective rate > 90%.

No medication is needed during treatment. It is also effective in treating diseases relating to insufficient enzyme for digestion of milk in stomach.

Example 20. Enhancing Growth of Hair

The apparatus of the present invention can be used for enhancing growth of hair when the following constituents are added into the simulated bio-spectrum generator:

silicon oxide $\geq$ 80%              ferric oxide $\geq$ 2%
vanadium oxide $\geq$ 2%              zinc oxide $\geq$ 2%

| | |
|---|---|
| titanium oxide ≥ 2% | boron nitride ≥ 2% |
| tungsten carbide ≥ 1% | magnesium oxide ≥ 2% |
| calcium oxide ≥ 5% | cerium oxide ≥ 2% |

This apparatus for treatment of baldness and enhancement of growth of hair is used as follows: daily irradiation of 1 - 2 times, 20 - 30 minutes a time is directed for at least one course (30 days) to sites of alopecia over the head, undersides of the feet, abdomen, lumbar, and kidney regions. This apparatus can be used alone or accompanied with prior irradiation local lotion of medications that enhance blood circulation in skin of skull, and stimulate growth of the hair.

Therapeutic effects: This apparatus is mainly used for treating alopecia seborrhoeica. It regulates endocrine secretion, enahnces metabolism, increases skin respiration, so that the subsided growth information and growth of hair roots can be restored.

It is also effective for other kinds of alopecia or even poliosis owing to its anti-senescent function (please refer to example 5).

Example 21. Treatment of Chloasma

The apparatus of example 19 can be used for treating chloasma in a way that irradiation is directed to face, umbilicus, abdomen, kidney region, undersides of the feet, and head one time a day, 20 - 30 minutes a time (30 times make a course).

When skin-protecting lotions or cosmetic measures are used, the facial region can be irradiated with the apparatus before or after the cosmetic measures. Umbilicus, abdomen, kidney region, undersides of the feet, and head can also be irradiated at the smae time. It is capable of accelerating the absorption of nutritive substances by the skin and improving blood supply and respiration of the skin locally, while regulating the endocrine system and eliminating impairment of circulation or nervous activities in the whole body, so as to remove the fundamental cause of chloasma internally through the organs, tissues, and the nervous system.

The apparatus of this example can be used for consmesis and health care. The effects are achieved through the improvement of general physical conditions and prevention of sensecence fundamentally.

Example 22. Applications in the Simulation of Bio-Informations

Certain informations of the living organism can be simulated with the present invention. For example, body temperature, pulse, heart rate, and the frequency, amplitude, or wave form of bio-physical parameters like ECG or EEG can be simulated by means of various apparatus for the purposes of training and induction, as well as treatment and health care of patients.

Example 22-1. Applications in the Simulation of Informations of Chinese "Qi-Gong" and Indian "Yuga"

It is known from recent studies and measurements that information of Chinese "Qi-Gong" and Indian "Yuga" is in the spectral range of um - mm. However, the changes and fluctuations of information emitted by those who are practicing Qi-Gong or Yuga are different. According to the results of these studies, the apparatus is capable of simulating informations of Chinese "Qi-Gong" and Indian "Yuga" using the present invention. This kind of apparatus will help train or induce the beginners, or will help the well-trained to enter the practicing state in non-quiet environments by simulating Qi-Gong or Yuga information. When the apparatus of the present invention is used to simulate Qi-Gong or Yuga information, the emitting layer of the simulated bio-spectrum generator should contain:

| | |
|---|---|
| aluminum oxide ≥ 85% | cerium oxide ≥ 1% |
| ferric oxide ≥ 5% | cobalt oxide ≥ 3% |
| chromium oxide ≥ 5% | silicon oxide ≥ 1% |

When the apparatus is supplied with an electrical power that pulsates at a frequency of 0 - 60 cycles/minute, the spectral information generated approaches that of Qi-Gong or Yuga, and can perform the function of training and health care.

Signals at a broad band or single frequency within the range of $\mu$m - mm, when changed both in amplitude and in frequency, will act like the information of Qi-Gong or Yuga.

Example 22-2.

According to the principle of the present invention and by using the apparatus of the present invention, the range of the spectrum is restricted to 0.6 - 30 $\mu$m. In this case, the simulated bio-spectrum generator contains magnesium fluoride ($MgF_2$) in 100%.

The simulated bio-spectrum of the apparatus of the present invention can also be made to contain mainly magnesium fluoride (50%), the other constituents can be materials with high chromaticity, high rate of radiation, and broad comprehensive spectrum. In this example, they are:

| | |
|---|---|
| magnesium fluoride ($MgF_2$) $\geq$ 50% | titanium oxide ($TiO_2$) $\geq$ 5% |
| nickel oxide (NiO) $\geq$ 5% | tin oxide ($SnO_2$) $\geq$ 1% |
| manganese oxide ($MnO_2$) $\geq$ 6% | (BN) $\geq$ 20% |

This apparatus is powered intermittently with a power source which works two seconds in every 10 seconds, so that a changing spectrum is generated.

Example 23. Application in Animals

The present apparatus is capable of enhancing the growth and development of animals, increasing hatchability of eggs, improving quality of semen, treating various diseases of the animals, and helping prolong the survival period of rare animals when the following constituents are added into the simulated bio-spectrum generator:

| | |
|---|---|
| chromium oxide $\geq$ 90% | ferric oxide $\geq$ 1% |
| chromium $\geq$ 1% | zinc oxide $\geq$ 1% |
| copper oxide $\geq$ 0.2% | cobalt oxide $\geq$ 0.2% |
| manganese oxide $\geq$ 0.5% | molybdenum oxide $\geq$ 0.1% |
| selenium oxide $\geq$ 1.5% | strontium oxide $\geq$ 0.1% |
| vanadium oxide $\geq$ 0.1% | aluminum oxide $\geq$ 0.2% |
| magnesium oxide $\geq$ 0.8% | silicon oxide $\geq$ 0.3% |
| germanium oxide $\geq$ 1% | lanthanium $\geq$ 0.1% |
| KI $\geq$ 0.1% | |

1. When this apparatus is used to irradiate the perinial and testis region of the stud stook once a day (20 - 60 minutes) with a surface power of 5 - 30 mW/cm$^2$ for 20 days, sperm activity is increased by 10 - 20%, sperm density by 5 - 14%, activity after freezing by 50%, qualified semen can be increased by over three times, and fertility rate is also increased significantly. The effect on the development of the fetus is apparent.

2. When this apparatus is used to irradiate the chickens 30 - 300 minutes a day with the skin temperature of the chicken being controlled to 31 - 37°C continuously or intermittently, body weight of 30-day chickens is increased and diarrhea of chickens is prevented effectively.

3. When the apparatus is used to irradiate the stud eggs, the hatching rate is increased. For this purpose, the eggs are fixed first, irradiation is directed thereto for 30 - 50 minutes, the surface temperature of the eggs is controlled to 36 - 37° C. The process is repeated once a day for 1 - 6 days. 1 - 3 times more irradiation before breaking of the shell can increase the hatching rate by 5 - 20% and can shift the time of outburst to 3 - 20 hours earlier than usual.

4. Application in the treatment of mastitis of cow is confirmed by irradiating to breast of the cow 30 - 50 minutes, 1 - 2 times a day, for a course (five days) or more. After treatment, infections can be controlled, inflammation can be eliminated, and milk production is restored.

5. Application in the treatment of gastroenteritis of animals has been proven. Irradiation to the region of the stomach and intestine should be directed 30 - 60 minutes a time, 1 - 3 times a day for a course (five days) or more. After treatment, inflammation can be eliminated, toxic substances in the gastroenteric tract can be neutralized and gastro-enteric functions are thus restored.

6. The apparatus can be used to treat trauma such as hematoma or sprain of joints. Irradiation to the sites of trauma or sprain, 20 - 40 minutes a time, 1 - 2 times a day for at least a course (four days) can eliminate hematoma, has the products of inflammation absorbed, and reduces sensitivity of the trauma and enhances regeneration and restoration.

Example 24. Applications in Plants

The apparatus of example 22 is capable of influencing the status of growth of plants, but small amounts of potassium, phosphorus, and nitrogen (each kind no more than 0.5%) must be added into the emitting layer of the simulated bio-spectrum generation and the ratio of ultraviolet and visible light in the radiation should also be increased. In this case, the ultraviolet ray can be obtained from a separate source, while the visible light is generated by the present apparatus itself.

The purposes of the application of the present apparatus in plants are:

1. To increase the resistance to adverse conditions, to increase cold resistance, drought resistance, and disease resistance, to improve quality, and to modify variety;

2 To increase rate of germination;

3. To shorten the period of growth of economic crops, to facilitate early ripening, and to increase production and benefits;

4. To influence gene information, to breed new variety, to alter the form and color of plants.

Taking seeds as an example: when seeds are germinating, the starch in endosperm is decomposed into monosaccharides and transported to the embryonic organs. Starch is hydrolysed into maltose by -amylase and -amylase, then decomposed into glucose by maltase to provide substances and energy for the growth of the embryo. The activity of amylase has direct influence on germination of the seeds and growth of the seedlings. The apparatus of the present invention are capable of increasing the total amylase by 100 more than the control group. The activities of peptidase, ascorbic acid oxidase, nitrate reductase, and lipoproteinase are also increased. Seedling respiration is increased after treatment of the seeds.

A. When tobacco seedlings were irradiated with the above mentioned apparatus once a day, 40 minutes a time for three consecutive days from a distance of 40 - 60 cm and with a surface intensity of 0.1 - 0.25 mW/cm$^2$, the irradiated tobacco seedlings became resistant to the tobacco mosaic virus, and their resistance to adverse condition and yield also increased.

B. Dry seeds of paddy rise were irradiated with the apparatus within 60 days before sowing under the following conditions: thickness of rice seeds was 1 - 1.5 cm; the simulated bio-spectrum generator was placed 25 - 60 cm apart from the seeds; duration of irradiation was 40 - 150 minutes perferably 100 minutes; surface temperature of the seeds was controlled to 41 - 49° C. After irradiation, rooting ability, tillering ability, shooting ability, drought resistance, disease resistance, quality of seeding were all increased, arid yield of rice was also increased.

The apparatus of the present invention can be widely applied to seedling culturing and seed treatment in agriculture, forestry and gardening.

Example 25. Application in Microorganisms

The importance of genes to living beings led to the development of bio-technology and gene engineering. Plants and animals are all composed of cells. There exists a nucleus in the cell and chromosomes in the nucleus. Nuclei of cells of different living beings have different numbers of chromosomes. There are 23 pairs of chromosomes in the nucleus of a human cell, four pairs in a fruit fly cell, seven pairs in a bean cell, but only one single unpaired chromosome in a bacterial cell. Chromosomes are composed of genes which are positioned on two DNA molecules entangled with each other. The specific chemical substances and their arrangements determine the information contained in the genes. DNA is the chemical protoplasm that directs the formation of proteins in the cell, and also performs the function of transporting the features of the original cell to a new cell or organism. The present invention provides a kind of simulated bio-spectrum that causes transition of energy levels of the molecules, atoms, and electrons in the cells. These changes can cast influence on the nucleus, chromosome, gene, or DNA directly, and will help in correcting the connection between chromosomes, and controlling the amount and arrangement of the chemical protoplasm. The present invention will help bio-technology and gene engineering directly or indirectly in the following aspects:

1. Irradiation of fungi with the apparatus of example 24, 1 - 3 irradiations at a dose of 10 - 2- mW/cm$^2$ for 30 minutes, can be made on stock plants, original seeds, or cultigens. It can increase functions of the hypha, decompose and absorb nutritions in the matrix and to differentiate fruiting bodies. As compared with the control group, there are tendencies to increase reproductivity, shorten the period of growth and increase yield.

2. Irradiation with the apparatus of example 24 can increase the yield and quality of antibiotics. When the simulated bio-spectrum is used to irradiate the cultures, the irradiation should be made with an intensity of 5-20 mW/cm$^2$ and at a temperature of 27 - 30° C for 1 - 3 times (40 minutes a time). Penicillin can be irradiated during the course of production for 40 minutes, the dose is 5 - 10 mW/cm$^2$, with ambient temperature of 25-27°C and pH value of 6.8 - 7.2.

The present invention shows a tendency to improve the respiration of the microorganism, and to make the absorption and transformation of oxygen to reach the utmost degree of metabolic activity. It can also be used to increase the quality of brewing.

Example 25-1. Direct Action on DNA

Erroneous connection of chromosome, arrangement, and amount of DNA can cause handicaps and diseases such as tumors or Down's Syndrome in human beings. The apparatus can perform the function of directly repairing and regulating DNA which is conducive to the relief of diseases. The emitting layer shoud contain the following compounds:

chromium oxide ≥ 80%       beryllium oxide ≥ 1%
copper oxide ≥ 2%          zinc oxide ≥ 3%
cobalt oxide ≥ 2%          niohium oxide ≥ 1%
calcium oxide ≥ 3%         strontium oxide ≥ 1%
silicon oxide ≥ 2%         selenium oxide ≥ 2%
molybdenum oxide ≥ 1%      ferric oxide ≥ 2%

Example 25-2. Enhancement of Miorobiologic Drug Manufacturing

The apparatus of example 25-1 can help in synthesizing insulin by DNA recombinaticn technique. The genes that produce insulin are first found out from the nucleus of the human beings and cut down with vestriction enzyme. Then, the plasmid in bacteria is also cut down and combined with the genes that produce insulin. The new combination is then put back into the bacteria, and the bacteria are put into the fermentation tank to propagate and hence produce insulin. When the present invention is used to irradiate the bacteria directly, or to make a bio-spectrum fermentation tank, the rate of successful DNA recombination, transplantation, and bacteria propagation can be increased, leading to the increase of insulin yield. This can also be used to synthesizing hormones and antibiotics.

Example 25-3. Construction of New Organic Tissue

When the gene recombination transplantation technique assisted by the apparatus of example 25-1 is applied to plants or animals, the speed of growth is accelerated, fruits of crops become larger, the crops are cold, pest, and drought resistant, the body weight and disease resistance of animals are increased. When using the present invention, the rate of successful gene recombination transplantation in plants or animals is increased because the present invention is capable of influencing the arrangement and amount of DNA. The apparatus of the present invention can be used to directly irradiate the cells or embryos after recombination of genes.

Example 26. Plastics and Textiles Which Can Radiate Simulated Bio-spectrum.

In order for the present invention to be applied widely, one or more monomers or compounds of chemical elements which can generate simulated bio-spectrum can be mixed with raw materials to make various kinds of plastics or textiles. The constituents that should be added are as follows:

zinc oxide ≥ 5%           ferric oxide ≥ 3%
silicon oxide ≥ 5%        germanium oxide ≥ 10%
thorium oxide ≥ 2%        chromium oxide ≥ 75%

The chemical elements of this example can be mixed into the raw material of plastics directly, into paints, dyes, enamels, or other coating materials which will be painted onto the surface of the plastic products. They can also be mixed into the textiles (cloth, artificial leather) to form a radiating surface membrane.

The plastics or textiles which can radiate simulated bio-spectrum can be used for health care of human body or animals, for crop breeding or seedling nursing as well as for microbiologic fermentation. The applications of the materials using different sources of energy are now described.

## 1. Electricity as Energy Source

Electric wires of certain resistance are embedded into plastics or textiles that contain the simulated bio-spectrum generating substances as described in example 26. The electric wires are designed to have certain heat generating power and cross sectional area like that used in the electric heating blanket. When being heated by electricity, this kind of plastic or textile can generate simulated bio-spectrum of certain intensity, which can enhance body fluid circulation and improve the function of the nervous system. It is beneficial to health when used for a long time. This kind of material can be made into electric heating blankets, local health bands, chair cushions for offices, cars, boats, or airplanes, matresses, beds for home use, and sporting goods. The heating temperature is controlled below 45°C.

## 2. Magnetic Field as Energy Source

Magnetic material can be added into the plastic or textile containing substances that generate the simulated bio-spectrum as described in example 26. The magnetic material can be various kinds of permanent magnets. Under the action of the magnetic field, the simulated bio-spectrum generated by the plastic or textile can be intensified. The dual action of the spectrum and magnetic field can enhance body fluid circulation and health care. This example can be used to make various kinds of mattresses or chair cushions, hats, and shoes, waist or knee bands, or to wrap strong magnetic materials to make various kinds of magneto-spectral health caring equipments.

## 3. Solar Energy as Heating Energy

Plastics or textiles, including plastic membrane, containing the simulated bio-spectrum generating substances as described in example 26 can be used to make large tents or rooms so that the plastics or textiles can be heated by solar energy and become the simulated bio-spectrum generator. This example can be used for seedling nursing, breeding, or low temperature culturing.

## 4. Body Heat or Ambient Heat as Energy Source

The plastics or textiles containing simulated bio-spectrum generating substances as described in example 26 can be used to make articles for daily use such as shoes, caps, waist bands, matresses or cushions, so that heat produced by human body ornatural heat of the environment can act as the energy source. Long term contact with such articles will assist blood circulation and heat preservation.

In other apparatuses not according to the present invention the chemical constituents can be adjusted appropriately. For example, the oxides can be substituted with fluorides, carbides, or nitrites. the mixture ratio of these constituents can also be readjusted, but at least one or a few of the elements in Table 1 or their compounds should be contained. When any one of the following elements or its compounds serves as the main constituent, it should account for no less than 50%:

chromium, magnesium, selenium, germanium, zinc, copper, aluminum, strontium, cerium, yttrium, calcium, zirconium, molybdenum, silicon, iron, vanadium.

The simulated bio-frequency spectrum generator can also contain the radiation source of gray body or near black body which consists of a plurality of ceramics, metals, or the combination of ceramic and metals.

The principle of determining the proportions of the above chemical constituent of determining radiation sources of the gray body or near black body mentioned above is that their spectrum should be as similar to the spectrum of the subject organism as possible. That is to say, the simulated bio-spectrum should overlap with the bio-spectrum; the more the overlap, the better. For complicated organisms such as human beings or animals, the spectrum to be simulated with the constituents in certain proportions should be as broad as possible. It is preferable to cover ultraviolet, visible light, infrared through millimeter waves, so that sufficient transitions of the molecules, atoms, and electrons can be elicited simultaneously.

The applicant finds whatever the proportions of the chemical elements may be, the key point is whether the spectral signal generated by the simulated bio-spectrum generator and the energy elicited by the electrons or the excited molecules can be utilized to achieve biological effects of beneficial regulation. A spectral range of 0.2 $\mu$m - 10 cm covers most of the bio-spectrum. A means for effectively adjusting and improving the development and survival of living organisms is provided through the application of the apparatus of the present invention. Many kinds of diseases can be treated with the apparatus of the present invention.

Table B.   Functions and Therapeutic Effects of the Apparatus
of the Present Invention

| Diseases | sites of treatment | Min./ time | times/ day | days/ course | therapeutic effects |
|---|---|---|---|---|---|
| common cold | face, vertebrae, undersides of feet | 20-60 | 1-4 | 1-6 | early stage: good; middle or late stage: fair effective rate ≥ 70% |
| German measles | lesion | 20-40 | 1-4 | 1-10 | relief of itching ≥ 70% |
| hepatitis | liver region, back, undersides of feet | 20-60 | 1-3 | 30-150 | effective rate ≥ 80% |
| dysentery | abdomen, undersides of feet | 20-50 | 1-4 | 1-10 | e.r. ≥ 80% |
| acute or chronic bronchitis | lungs, bronchus, back | 20-60 | 1-2 | 3-30 | e.r. ≥ 60% |
| asthma | lungs, bronchus, back | 20-60 | 1-3 | 30-90 | e.r. ≥ 60% |
| pneumonia | lungs, bronchus, back | 20-60 | 1-3 | 2-7 | e.r. ≥ 80% |
| rheumatic fever | lungs, bronchus, back | 20-50 | 1-2 | 10-20 | remission rate ≥ 40% |
| rheumatic heart disease | chest, joint | 20-50 | 1-2 | 10-60 | r.r. ≥ 60% |
| hypertension | chest, | 20-40 | 1-2 | 30 | effective |

| | vertebra,<br>head,<br>undersides<br>of feet | | | | rate ≥ 90% |
|---|---|---|---|---|---|
| coronary<br>heart<br>disease | chest,<br>head, feet | 20-40 | 1-2 | 30 | e.r. ≥ 90% |
| viral<br>myocarditis | chest,<br>head, feet | 20-40 | 1-2 | 30 | e.r. ≥ 85% |
| Raynaud's<br>disease | chest,<br>head, feet | 20-40 | 1-2 | 30 | e.r. ≥ 70% |
| arrhythmia | chest,<br>head, feet | 20-40 | 1-2 | 5-30 | e.r. ≥ 70% |
| heart<br>failure | chest,<br>head, feet | 20-40 | 1-2 | 5-30 | e.r. ≥ 80% |
| acute and<br>chronic<br>gastritis | gastric<br>region,<br>abdomen | 20-40 | 1-2 | 5-30 | e.r. ≥ 85% |
| peptic ulcer | gastric<br>region,<br>abdomen | 20-40 | 1-2 | 30 | e.r. ≥ 90% |
| chronic<br>colitis | gastric<br>region,<br>abdomen | 20-40 | 1-2 | 30 | e.r. ≥ 90% |
| cirrhosis of<br>liver | liver,<br>abdomen,<br>back, feet | 20-40 | 1-3 | 30 | e.r. ≥ 70% |
| acute and<br>chronic<br>pancretitis | lesion,<br>abdomen,<br>lumbar region | 20-40 | 1-3 | 2-30 | e.r. ≥ 65% |
| gastro-<br>enteric<br>neurosis | stomach,<br>abdomen,<br>undersides<br>of feet | 20-60 | 1-3 | 3-30 | e.r. ≥ 70% |
| leukemia | whole body,<br>chest, | 20-60 | 1-3 | 30-90 | e.r. ≥ 30% |

| | | | | | |
|---|---|---|---|---|---|
| | spleen, abdomen, vertebra, feet | | | | |
| diabetes | region of islets of Langerhans abdomen, lumbar region, feet | 20-60 | 1-3 | 3-90 | e.r. ≥ 60% |
| disturbance of acid base equilibrium | chest, abdomen, vertebra, feet | 20-60 | 1-3 | 5-30 | e.r. ≥ 60% |
| obesity | abdomen, back, feet | 20-60 | 1-3 | 30 | remission rate ≥ 60% |
| malnutrition | abdomen, stomach, kidney region, feet | 20-60 | 1-3 | 5-30 | r.r. ≥ 50% |
| beri-beri | lesion | 20-60 | 1-3 | 7-30 | r.r. ≥ 60% |
| nephrosis syndrome | kidney region, feet, abdomen, whole body | 20-60 | 1-3 | 30 | r.r. ≥ 75% |
| uremia | kidney region, feet, abdomen, whole body | 20-60 | 1-3 | 30 | r.r. ≥ 60% |
| alcoholism | liver, abdomen, stomach, whole body | 20-60 | 1-3 | 5-30 | effective rate ≥ 65% |
| narcotics abstinence | head, feet abdomen, stomach, whole body | 20-60 | 1 | 30 | e.r. ≥ 65% |
| food poisoning | liver, abdomen, | 60 | 1-3 | 1-7 | e.r. ≥ 70% |

| stomach, whole body | | | | | |
|---|---|---|---|---|---|
| remission of radiation sickness | whole body | 60 | 1-2 | 30 | e.r. ≥ 65% |
| intensification of anesthesia | site of injection of anesthetics | 5-20 | 1 | 1 | e.r. ≥ 70% |
| sedation | head, feet abdomen | 20-60 | 1-2 | 1-10 | e.r. ≥ 80% |
| hypnogenesis | head, feet, stomach, abdomen | 20-50 | 1 | 30 | e.r. ≥ 70% |
| analgesia | lesion | 20-60 | 1-3 | 30 | e.r. ≥ 80% |
| closed wound | wound | 20-40 | 1-2 | 2-10 | e.r. ≥ 85% |
| open wound | wound | 20-40 | 1-3 | 2-10 | e.r. ≥ 90% |
| quick healing of surgical wound | wound | 20-40 | 1-3 | 3-5 | e.r. ≥ 90% |
| burns | lesion | 20-40 | 1-3 | 3-5 | e.r. ≥ 90% |
| frostbite | lesion | 20-40 | 1-3 | 3-5 | e.r. ≥ 90% |
| poisionous snake bite | lesion | 60 | 1-3 | 10 | e.r. ≥ 80% |
| pyemia | lesion | 30-50 | 1-3 | 10 | e.r. ≥ 80% |
| furuncle | lesion | 30-50 | 1-3 | 3-10 | e.r. ≥ 80% |
| carbuncle | lesion | 30-50 | 1-3 | 3-10 | e.r. ≥ 85% |
| cellulitis | lesion | 30-50 | 1-3 | 3-10 | e.r. ≥ 85% |
| lymphangioitis | lesion | 30-50 | 1-3 | 3-10 | e.r. ≥ 85% |
| erysipelas | lesion | 30-50 | 1-3 | 3-10 | e.r. ≥ 85% |
| paronychia | lesion | 30-50 | 1-3 | 2-8 | e.r. ≥ 85% |
| peritendinitis | lesion | 30-50 | 1-2 | 30 | e.r. ≥ 60% |
| bone tumors | whole body | 20-60 | 1-3 | 5-30 | remission |

| | lesion, with healing of surgical wound | | | | |
|---|---|---|---|---|---|
| damage of peripheral nerves | lesion | 20-40 | 1-3 | 10-30 | effective rate ≥ 60% |
| difficulty in urination | perinium, abdomen, kidney region | 20-40 | 1-3 | 5-20 | e.r. ≥ 60% |
| retention of urine | perinium, abdomen, kidney region | 20-40 | 1-3 | 5-20 | e.r. ≥ 60% |
| swelling of scrotum | lesion | 20-40 | 1-3 | 5-30 | e.r. ≥ 60% |
| cyctitis | perinium, bladder | 20-40 | 1-3 | 5-30 | e.r. ≥ 70% |
| prostatitis | perinium, lower abdomen | 20-40 | 1-3 | 5-30 | e.r. ≥ 80% |
| renal hyper-tension | kidney region, feet, whole body | 20-40 | 1-3 | 30-60 | e.r. ≥ 80% |
| anti-inflam-mation and analgesia of gall stone and kidney stone | lesion | 20-40 | 1-3 | 3-15 | remission |
| infertility or sterility | whole body, perinium, abdomen, uterus, feet, verte-brae | 20-40 | 1-3 | 5-30 | effective rate ≥ 60% |
| disturbance in sexual | whole body, perinium, | 20-40 | 1-3 | 5-30 | e.r. ≥ 60% |

29

function

| | | | | | |
|---|---|---|---|---|---|
| care of premature infant | whole body | 15-60 | 1-3 | 3-20 | e.r. ≥ 60% |
| hepatitis accompanied by pregnancy | whole body, liver region, feet | 20-40 | 1-2 | 20-80 | e.r. ≥ 60% |
| puerpural infection | lesion | 20-40 | 1-3 | 5-15 | e.r. ≥ 70% |
| cervicitis | lesion | 20-40 | 1-3 | 30 | e.r. ≥ 85% |
| vaginitis | lesion | 20-40 | 1-3 | 5-30 | e.r. ≥ 85% |
| pelvic infection | perinium | 20-40 | 1-3 | 30 | e.r. ≥ 85% |
| metropathia hemorrhagica | perinium, uterine projection area | 20-40 | 1-3 | 30 | remission |
| lactation menopause syndrome | lesion, feet, abdomen | 20-40 | 1-3 | 5-30 | effective rate ≥ 70% |
| premenstrual anxiety | abdomen, feet | 20-40 | 1-3 | 5-30 | e.r. ≥ 90% |
| climateric syndrome | whole body | 20-40 | 1-3 | 30 | e.r. ≥ 45% |
| menopause | whole body, abdomen | 20-40 | 1-3 | 30 | e.r. ≥ 50% |
| menorrhalgia | abdomen | 20-40 | 1-3 | 30 | e.r. ≥ 50% |
| breast tumor | lesion, whole body | 30-60 | 1-2 | 30 | remission |
| gas gangrene | lesion | 40 | 1-3 | 10 | remission |
| mastitis | lesion | 30-50 | 1-2 | 2-10 | effective rate ≥ 70% |
| proliferation of lobuli | lesion | 30-50 | 1-2 | 30 | e.r. ≥ 70% |

mammae

| | | | | | |
|---|---|---|---|---|---|
| hernia | lesion | 30-50 | 1-2 | 30 | e.r. ≥ 60% |
| peritonitis | lesion | 30-50 | 1-2 | 30 | e.r. ≥ 70% |
| fistula or abscess of anus or intestine | lesion | 30-50 | 1-2 | 30 | e.r. ≥ 90% |
| fissura ani | lesion | 30-50 | 1-2 | 2-20 | e.r. ≥ 90% |
| hemorrhoid | lesion | 30-50 | 1-2 | 2-20 | e.r. ≥ 90% |
| cholecystitis | lesion | 30-50 | 1-2 | 2-20 | e.r. ≥ 70% |
| angiitis | lesion | 30-50 | 1-2 | 30 | e.r. ≥ 30% |
| varicose of vein of lower extremity | lesion | 30-50 | 1-2 | 30 | remission |
| chronic ulcus cruris | lesion | 30-50 | 1-2 | 30 | effective rate ≥ 80% |
| quick healing of plastic operation | lesion | 20-40 | 1-2 | 2-7 | e.r ≥ 95% |
| elimination of repelling reaction | lesion | 20-60 | 1-3 | 2-10 | e.r. ≥ 95% |
| quick healing of fracture | lesion | 20-60 | 1-3 | 2-10 | e.r. ≥ 85% |
| damage of meniscus | lesion | 20-60 | 1-3 | 5-20 | e.r. ≥ 75% |
| enhancement of healing of replantation of extremities | lesion | 20-60 | 1-3 | 3-10 | e.r. ≥ 95% |
| purulent osteomyelitis | lesion | 20-60 | 1-3 | 20-40 | e.r. ≥ 80% |
| rheumatoid arthritis | lesion | 20-60 | 1-3 | 30-60 | e.r. ≥ 80% |
| arthritis | lesion | 20-60 | 1-3 | 30-60 | e.r. ≥ 90% |

| osteoporosis | lesion, whole body | 20-60 | 1-3 | 30-60 | e.r. ≥ 70% |
|---|---|---|---|---|---|
| sequalae | lesion | 20-60 | 1-3 | 30-60 | remission |
| pain in neck, shoulder, or lumbar region | lesion | 20-60 | 1-3 | 3-30 | effective rate ≥ 95% |
| periarthritis of shoulder joint | lesion | 20-60 | 1-3 | 3-30 | e.r. ≥ 95% |
| epichodylitis of humerus | lesion | 20-60 | 1-3 | 3-30 | e.r. ≥ 95% |
| vomiting of infant | stomach, abdomen | 15-20 | 1-2 | 5 | e.r. ≥ 90% |
| scleroderma neonatorum | abdomen, perinium | 15-20 | 1-2 | 5 | e.r. ≥ 90% |
| subcutaneous gangrene of newborn infant | lesion | 15-20 | 1-2 | 7 | e.r. ≥ 80% |
| septicemia of newborn infant | whole body | 15-20 | 1-2 | 7 | remission |
| tetanus of newborn infant | whole body | 15-30 | 1-2 | 10 | remission |
| hemolysis of newborn infant | whole body | 15-30 | 1-2 | 10 | remission |
| infantile diarrhea | abdomen | 15-30 | 1-2 | 2-7 | effective rate ≥ 90% |
| inflammation infant buccal cavity | inside or outside of lesion | 15-20 | 1 | 2-10 | e.r. ≥ 70% |
| acute respiratory infection of infant | chest, accompanied with medication | 15-20 | 1 | 3-7 | e.r. ≥ 95% |
| infantile pneumonia | chest, accompanied with medic- | 15-20 | 1 | 3-7 | e.r. ≥ 95% |

ation

| | | | | | |
|---|---|---|---|---|---|
| infantile nephrosis | kidney region, feet, whole body | 15-30 | 1 | 10 | e.r. ≥ 85% |
| infantile tuberculosis | chest, spleen region, feet | 15-30 | 1 | 20 | e.r. ≥ 70% |
| mild disturbance of cerebral function of infant | head, feet, vertebrae | 15-30 | 1 | 20 | e.r. ≥ 70% |
| incontinence of urine | perinium, lumbar and kidney region | 15-30 | 1-2 | 3-7 | e.r. ≥ 95% |
| immuno-deficiency | whole body | 15-30 | 1-2 | 20 | remission |
| paralysis | whole body | 60 | 1-2 | 30 | remission |
| facial neuritis | face | 30-50 | 1-2 | 3-15 | effective rate ≥ 80% |
| trigeminal neuralgia | lesion | 30-50 | 1-2 | 3-15 | e.r. ≥ 85% |
| sciatica | lesion | 30-50 | 1-2 | 3-15 | e.r. ≥ 85% |
| epilepsy | head, brain, feet, cervical vertebrae | 30-50 | 1-2 | 3-30 | e.r. ≥ 90% |
| sequelae of brain damage | lesion | 30-50 | 1-2 | 3-30 | e.r. ≥ 80% |
| psychosis and hysteria | head, whole body | 30-50 | 1-2 | 30 | e.r. ≥ 70% |
| neurosis | head, vertebra, abdomen, | 30-50 | 1-2 | 3-30 | e.r. ≥ 70% |

| | | | | | |
|---|---|---|---|---|---|
| | feet | | | | |
| neurasthenia | head, vertebra, abdomen, feet | 30-50 | 1-2 | 3-30 | e.r. ⩾ 70% |
| hysteria | head, vertebra, abdomen, feet | 30-50 | 1-2 | 3-30 | e.r. ⩾ 70% |
| schizophrenia | head, vertebra, abdomen, feet | 30-50 | 1-2 | 3-30 | e.r. ⩾ 70% |
| senile dementia | brain, whole body | 20-40 | 1-2 | 30 | remission |
| herpes simplex | lesion | 20-40 | 1-3 | 5-10 | effective rate ⩾ 95% |
| herpes zoster | lesion | 20-40 | 1-3 | 5-10 | e.r. ⩾ 95% |
| pustule | lesion | 20-40 | 1-3 | 5-10 | e.r. ⩾ 95% |
| folliculitis | lesion | 20-40 | 1-3 | 5-10 | e.r. ⩾ 95% |
| syphilis | lesion | 30-60 | 1-2 | 30 | remission |
| tinea | lesion | 30-60 | 1-2 | 10-30 | effective rate ⩾ 85% |
| coccidioido-mycosis | lesion | 30-60 | 1-2 | 10-30 | e.r. ⩾ 85% |
| aspergillosis | lesion | 30-60 | 1-2 | 10-30 | e.r. ⩾ 90% |
| scabies | lesion | 30-60 | 1-2 | 10-30 | e.r. ⩾ 90% |
| allergic dermatitis, eczema | lesion | 30-60 | 1-2 | 10-30 | e.r. ⩾ 90% |
| pruritus | lesion | 30-60 | 1-2 | 10-30 | e.r. ⩾ 90% |
| softening of corn | lesion | 30-60 | 1-2 | 10-30 | e.r. ⩾ 90% |
| actinic dermatitis | lesion | 30-60 | 1-2 | 10-30 | e.r. ⩾ 80% |

| frostbite | lesion | 30-60 | 1-2 | 2-7 | e.r. ≥ 98% |
|---|---|---|---|---|---|
| cold injury | lesion | 30-60 | 1-2 | 2-7 | e.r. ≥ 90% |
| psoriasis | lesion | 30-60 | 1-2 | 10-30 | e.r. ≥ 80% |
| pityriasis rosea | lesion | 30-60 | 1-2 | 10-30 | e.r. ≥ 70% |
| erythema multiforme | lesion | 30-60 | 1-2 | 10-30 | e.r. ≥ 90% |
| allergic noclular vasculitis of skin | lesion | 30-60 | 1-2 | 10-30 | e.r. ≥ 70% |
| Behcet's disease | lesion | 30-60 | 1-2 | 30 | e.r. ≥ 70% |
| lupus erythematosus | lesion | 30-60 | 1-2 | 30 | remission |
| scleroderma | lesion | 30-60 | 1-2 | 30 | remission |
| vitiligo | lesion | 30-60 | 1-2 | 30 | remission |
| chloasma | lesion | 30-60 | 1-2 | 30 | effective rate ≥ 70% |
| acne | lesion | 30-60 | 1-2 | 30 | e.r. ≥ 70% |
| dermatitis seborrhaeica | lesion | 20-50 | 1-2 | 30 | e.r. ≥ 80% |
| alopecia | lesion, accompanied with medication | 20-50 | 1-2 | 30 | e.r. ≥ 85% |
| osmidrosis | axilla, whole body | 20-50 | 1-2 | 30 | remission |
| anhyolrosis | axilla, undersides of feet, whole body | 20-50 | 1-2 | 30 | remission |
| keloid | lesion | 20-40 | 1-3 | 30 | remission |
| acute hemorragic | both eyes | 20-40 | 1-3 | 1-7 | effective rate ≥ 80% |

conjunctivitis

| | | | | | |
|---|---|---|---|---|---|
| ciliary blepharitis | lesion | 20-40 | 1-3 | 5-30 | e.r. ≥ 90% |
| conjunctivitis | lesion | 20-40 | 1-3 | 3-15 | e.r. ≥ 90% |
| scleritis | lesion | 20-40 | 1-2 | 5-20 | e.r. ≥ 60% |
| glaucoma | lesion | 20-40 | 1-2 | 30 | remission |
| juvenile myopia | lesion | 20-40 | 1-2 | 30 | effective rate ≥ 90% |
| presbyopia | lesion | 20-40 | 1-2 | 30 | remission |
| rhinitis | lesion | 20-40 | 1-2 | 30 | effective rate ≥ 80% |
| atrophic and allergic sinusitis | lesion | 20-40 | 1-2 | 30 | e.r. ≥ 80% |
| pharyngitis and laryngitis | lesion | 20-40 | 1-2 | 30 | e.r. ≥ 80% |
| tonsilitis | lesion | 20-40 | 1-2 | 30 | e.r. ≥ 80% |
| otitis media | lesion | 20-40 | 1-2 | 5-30 | e.r. ≥ 85% |
| inflammation or furuncle of external auditory canal | lesion | 20-40 | 1-2 | 3-10 | e.r. ≥ 85% |
| toothache, hemorrhage | lesion | 20-40 | 1-2 | 3-20 | e.r. ≥ 70% |
| pulpitis | lesion | 20-40 | 1-2 | 30 | e.r. ≥ 60% |
| AIDS | whole body, chest, liver, abdomen, spleen, lymph, feet, head | 30-60 | 1-3 | 30 | e.r. ≥ 80% |
| tumors | lesions, whole body | 30-60 | 1-3 | 30 | remission |
| protection | whole body | 20-60 | 1-3 | 1-30 | effective |

| | | | | | |
|---|---|---|---|---|---|
| of radiation | | | | | rate ≥ 75% |
| delay of senility | whole body | 20-45 | 1-2 | 30 | e.r. ≥ 70% |
| human health care | whole body | 20-60 | 1-3 | 30 | e.r. ≥ 95% |
| elimination of fatigue, restoration of physical ability | kidney region, feet, abdomen, head | 20-50 | 1-2 | 5-20 | e.r. ≥ 90% |
| thyroidism | thyroid region, abdomen, stomach, feet, liver, whole body | 20-50 | 1-2 | 30 | e.r. ≥ 70% |

## Claims

1. An apparatus for regulating and improving the status of growth and survival of living organisms, said apparatus comprising :

a) an energy source (1) ;
b) an energy transducing means (2) which converts the energy provided by said energy source (1) into thermal or magnetic energy ; and
c) a radiation generating means (4) energized by said thermal or magnetic energy provided by said energy transducing means (2) for generating a electromagnetic radiation so-called simulated bio-spectrum ;
characterized in that said radiation generating means (4) is constituted by a composition comprising several chemical elements or compounds mixed in a predetermined mixture ratio, said composition and relative mixture ratio being selected among the following ones:

(a)

| | |
|---|---|
| chromium oxide ≥ 95% | selenium oxide ≥ 1% |
| chromium ≥ 0.8% | germanium oxide ≥ 0.8% |
| zinc oxide ≥ 0.5% | ferric oxide ≥ 0.5% |
| magnesium oxide ≥ 0.2% | copper oxide ≥ 0.1% |
| cobalt oxide ≥ 0.1% | manganese oxide ≥ 0.1% |
| molybdenum oxide ≥ 0.1% | strontium oxide ≥ 0.1% |
| vanadium oxide ≥ 0.1% | aluminum oxide ≥ 0.1% |
| silicon oxide ≥ 0.1 | lanthanium ≥ 0.1% |
| magnesium fluoride ≥ 0.1% | |

(b)

| | |
|---|---|
| chromium oxide ≥ 95% | ferric oxide ≥ 0.5% |
| chromium ≥ 0.8% | zinc oxide ≥ 0.4% |
| copper oxide ≥ 0.1% | cobalt oxide ≥ 0.1% |
| manganese oxide ≥ 0.1% | molybdenum oxide ≥ 0.1% |
| selenium oxide ≥ 0.7% | strontium oxide ≥ 0.1% |
| vanadium oxide ≥ 0.1% | aluminum oxide ≥ 0.1% |

37

| | |
|---|---|
| magnesium oxide $\geq$ 0.1% | silicon oxide $\geq$ 0.1% |
| chromium oxide $\geq$ 0.8% | lanthanium $\geq$ 0.1% |
| $BO_2 \geq$ 0.1% | $MgF_2 \geq$ 0.1% |

(c)

| | |
|---|---|
| chromium oxide $\geq$ 93% | ferric oxide $\geq$ 0.5% |
| chromium $\geq$ 0.8% | zinc oxide $\geq$ 0.5% |
| copper oxide $\geq$ 0.1% | cobalt oxide $\geq$ 0.1% |
| manganese oxide $\geq$ 0.1% | molybdenum oxide $\geq$ 0.1% |
| selenium oxide $\geq$ 1% | strontium oxide $\geq$ 0.1% |
| vanadium oxide $\geq$ 0.1% | aluminum oxide $\geq$ 0.1% |
| magnesium oxide $\geq$ 0.1% | silicon oxide $\geq$ 0.1% |
| germanium oxide $\geq$ 1% | lanthanium oxide $\geq$ 0.1% |
| $MgF_2 \geq$ 0.1% | |

and suitable amounts of lithium, potassium, or titanium not more than 0.2% for each element.

(d)

| | |
|---|---|
| chromium oxide $\geq$ 94% | ferric oxide $\geq$ 0.5% |
| chromium $\geq$ 0.8% | zinc oxide $\geq$ 0.7% |
| copper oxide $\geq$ 0.2% | cobalt oxide $\geq$ 0.2% |
| manganese oxide $\geq$ 0.3% | molybdenum oxide $\geq$ 0.1% |
| selenium oxide $\geq$ 0.9% | strontium oxide $\geq$ 0.1% |
| vanadium oxide $\geq$ 0.1% | aluminum oxide $\geq$ 0.1% |
| magnesium oxide $\geq$ 0.1% | silicon oxide $\geq$ 0.1% |
| germanium oxide $\geq$ 0.8% | lanthanium oxide $\geq$ 0.1% |
| $CaCO_3 \geq$ 0.1% | |

(e)

| | |
|---|---|
| chromium oxide $\geq$ 94% | ferric oxide $\geq$ 0.5% |
| chromium $\geq$ 0.8% | zinc oxide $\geq$ 0.5% |
| copper oxide $\geq$ 0.2% | cobalt oxide $\geq$ 0.2% |
| manganese oxide $\geq$ 0.3% | molybdenum oxide $\geq$ 0.1% |
| selenium oxide $\geq$ 0.9% | strontium oxide $\geq$ 0.1% |
| vanadium oxide $\geq$ 0.1% | aluminum oxide $\geq$ 0.1% |
| magnesium $\geq$ 0.1% | silicon oxide $\geq$ 0.1% |
| germanium oxide $\geq$ 0.8% | lanthanium oxide $\geq$ 0.1% |
| $KI \geq$ 0.1% | $BO_2 \geq$ 0.1% |
| $CaCO_3 \geq$ 0.1% | $MgF_2 \geq$ 0.1% |

(f)

| | |
|---|---|
| chromium oxide $\geq$ 94% | ferric oxide $\geq$ 0.5% |
| chromium $\geq$ 0.8% | zinc oxide $\geq$ 0.5% |
| copper oxide $\geq$ 0.1% | cobalt oxide $\geq$ 0.1% |
| manganese oxide $\geq$ 0.1% | molybdenum oxide $\geq$ 0.1% |
| selenium oxide $\geq$ 0.9% | strontium oxide $\geq$ 0.1% |
| vanadium oxide $\geq$ 0.1% | aluminum oxide $\geq$ 0.1% |
| magnesium oxide $\geq$ 0.1% | silicon oxide $\geq$ 0.1% |
| germanium oxide $\geq$ 0.8% | lanthanium oxide $\geq$ 0.1% |

and a small amount of lithium, potassium, or sodium not more than 0.5%.

(g)

| | |
|---|---|
| chromium oxide ≥ 92% | ferric oxide ≥ 0.5% |
| chromium ≥ 0.8% | zinc oxide ≥ 0.5% |
| copper oxide ≥ 0.1% | cobalt oxide ≥ 0.1% |
| manganese oxide ≥ 0.2% | molybdenum oxide ≥ 1% |
| selenium oxide ≥ 1% | strontium oxide ≥ 0.1% |
| vanadium oxide ≥ 0.1% | lanthanium ≥ 0.1% |
| KI> ≥ 0.1% | |

and a suitable amount of titanium or boron, each of which in an amount not more than 0.2%.

(h)

| | |
|---|---|
| chromium oxide ≥ 90% | ferric oxide ≥ 1% |
| chromium ≥ 1% | zinc oxide ≥ 1% |
| copper oxide ≥ 0.2% | cobalt oxide ≥ 0.2% |
| manganese oxide ≥ 0.5% | molybdenum oxide ≥ 0.1% |
| selenium oxide ≥ 1.5% | strontium oxide ≥ 0.1% |
| vanadium oxide ≥ 0.1% | aluminum oxide ≥ 0.2% |
| magnesium oxide ≥ 0.8% | silicon oxide ≥ 0.3% |
| germanium oxide ≥ 1% | lanthanium ≥ 0.1% |
| KI ≥ 0.1% | |

(i)

| | |
|---|---|
| selenium oxide ≥ 5% | germanium oxide ≥ 5% |
| silicon oxide ≥ 50% | thorium oxide ≥ 4% |
| cerium oxide ≥ 5% | zirconium oxide ≥ 15% |
| boron nitride ≥ 10% | titanium carbide ≥ 3% |
| tungsten carbide ≥ 3% | platinum ≥ 0.01% |

(j)

| | |
|---|---|
| aluminum oxide ≥ 85% | ferric oxide≥ 3% |
| cobalt oxide ≥ 2% | titanium oxide ≥ 3% |
| silicon oxide ≥ 3% | hafnium oxide ≥ 2% |
| platinum ≥ 0.01% | |

(k)

| | |
|---|---|
| yttrium oxide ≥ 5% | ReCaMnFeOx ≥ 88% |
| silicon oxide ≥ 5% | |

(l)

| | |
|---|---|
| chromium oxide ≥ 95% | chromium ≥ 1% |
| zinc oxide ≥ 1% | silicon oxide ≥ 1% |
| cerium oxide ≥ 1% | |

(m)

| | |
|---|---|
| titanium oxide ≥ 90% | zirconium oxide ≥ 5% |
| silicon oxide ≥ 1% | ferric oxide ≥ 1.5% |
| zinc oxide ≥ 1% | copper oxide ≥ 1% |
| beryllium oxide ≥ 0.5% | |

(n)

| | |
|---|---|
| ferric oxide ≥ 20% | zirconium oxide ≥ 75% |

| | |
|---|---|
| silicon oxide ≥ 2% | zinc oxide ≥ 1% |
| niobium oxide ≥ 2% | |

(o)

| | |
|---|---|
| silicon oxide ≥ 80% | ferric oxide ≥ 2% |
| vanadium oxide ≥ 2% | zinc oxide ≥ 2% |
| titanium oxide ≥ 2% | boron nitride ≥ 2% |
| tungsten carbide ≥ 1% | magnesium oxide ≥ 2% |
| tungsten oxide ≥ 5% | cerium oxide ≥ 2% |

(p)

| | |
|---|---|
| aluminum oxide ≥ 80% | cerium oxide ≥ 1% |
| ferric oxide ≥ 5% | cobalt oxide ≥ 3% |
| chromium oxide ≥ 5% | silicon oxide ≥ 1% |

2. The apparatus according to claim 1, wherein said radiation generating means comprises a non-metal substrate; a layer composed of said composition and plated onto the surface of said substrate, and an electrothermal converting means provided within said substrate.

3. The apparatus according to claim 1, wherein said radiation generating means comprises a non-metal substrate; a conducting membrane of metal oxides plated onto the surface of said substrate to form an electrothermal energy converting means; and a layer composed of said composition coated on the surface of said conducting membrane.

4. The apparatus according to claim 1, wherein said radiation generating means comprises a non-metal substrate containing therein said composition and an electrothermal energy converting means.

5. The apparatus according to claim 1, wherein said radiation generating means comprises a non-metal substrate containing therein said composition, and electrically conducting substances for converting electrical energy to thermal energy.

6. The apparatus according to claim 1, wherein said radiation generating means comprises a metal substrate, a layer composed of said composition and plated directly onto the surface of the substrate or coated onto it in the form of enamel pulp and an electrothermal energy converting means provided within said substrate.

7. The apparatus according to claim 1, wherein said radiation generating means comprises a substrate made from high temperature resistant glass containing therein said composition and a layer of electrically conducting membrane made from metal oxides and formed on the surface of the substrate for converting electrical energy to thermal energy.

8. The apparatus according to claim 1, wherein said energy generating means is a source of magnetic field; and wherein said radiation generating means comprises a substrate made from magnetic material and a coating composed of said composition and painted onto said substrate.

**Patentansprüche**

1. Vorrichtung zur Regelung und Verbesserung des Wachstums- und Lebenserhaltungszustands lebender Organismen, wobei diese Vorrichtung umfaßt:

   a) eine Energiequelle (1);

   b) ein energieumwandelndes Mittel (2), welches die von der Energiequelle (1) bereitgestellte Energie in thermische oder magnetische Energie umwandelt; und

   c) ein strahlungserzeugendes Mittel(4), welches durch die thermische oder magnetische Energie angetrieben wird, die von dem energieumwandelnden Mittel (2) bereitgestellt wird, zum Erzeugen einer elektromagnetischen Strahlung, die als simuliertes Biospektrum bezeichnet wird;

**dadurch gekennzeichnet**, daß das strahlungserzeugende Mittel (4) aus einer Zusammensetzung gebildet ist, welche mehrere chemische Elemente oder Verbindungen umfaßt, die in einem vorgegebenen Mischungsverhältnis vermischt sind, wobei die Zusammensetzung und das relative Mischungsverhältnis aus den folgenden ausgewählt sind:

(a)

| | |
|---|---|
| Chromoxid ≥ 95 % | Selenoxid ≥ 1 % |
| Chrom ≥ 0,8 % | Germaniumoxid ≥ 0,8 % |
| Zinkoxid ≥ 0,5 % | Eisen(III)-Oxid ≥ 0,5 % |
| Magnesiumoxid ≥ 0,2 % | Kupferoxid ≥ 0,1 % |
| Cobaltoxid ≥ 0,1 % | Manganoxid ≥ 0,1 % |
| Molybdänoxid ≥ 0,1 % | Strontiumoxid ≥ 0,1 % |
| Vanadiumoxid ≥ 0,1 % | Aluminiumoxid ≥ 0,1 % |
| Siliciumoxid ≥ 0,1 | Lanthan ≥ 0,1 % |
| Magnesiumfluorid ≥ 0,1 % | |

(b)

| | |
|---|---|
| Chromoxid ≥ 95% | Eisen(III)-oxid ≥ 0,5% |
| Chrom ≥ 0,8% | Zinkoxid ≥ 0,4% |
| Kupferoxid ≥ 0,1% | Cobaltoxid ≥ 0,1% |
| Manganoxid ≥ 0,1% | Molybdänoxid ≥ 0,1% |
| Selenoxid ≥ 0,7% | Strontiumoxid ≥ 0,1% |
| Vanadiumoxid ≥ 0,1% | Aluminiumoxid ≥ 0,1% |
| Magnesiumoxid ≥ 0,1% | Siliciumoxid ≥ 0,1% |
| Chromoxid ≥ 0,8% | Lanthan ≥ 0,1% |
| $BO_2$ ≥ 0,1% | $MgF_2$ ≥ 0,1% |

c)

| | |
|---|---|
| Chromoxid ≥ 93% | Eisen(III)-oxid ≥ 0,5% |
| Chrom ≥ 0,8% | Zinkoxid ≥ 0,5% |
| Kupferoxid ≥ 0,1% | Cobaltoxid ≥ 0,1% |
| Manganoxid ≥ 0,1% | Molybdänoxid ≥ 0,1% |
| Selenoxid ≥ 1 % | Strontiumoxid ≥ 0,1% |
| Vanadiumoxid ≥ 0,1% | Aluminiumoxid ≥ 0,1% |
| Magnesiumoxid ≥ 0,1% | Siliciumoxid ≥ 0,1% |
| Germaniumoxid ≥ 1% | Lanthanoxid ≥ 0,1% |
| $MgF_2$ ≥ 0,1% | |

und geeignete Mengen von Lithium, Kalium oder Titan, die nicht mehr als 0,2% für jedes Element betragen.

d)

| | |
|---|---|
| Chromoxid ≥ 94% | Eisen(III)-oxid ≥ 0,5% |
| Chrom ≥ 0,8% | Zinkoxid ≥ 0,7% |
| Kupferoxid ≥ 0,2% | Cobaltoxid ≥ 0,2% |
| Manganoxid ≥ 0,3% | Molybdänoxid ≥ 0,1% |
| Selenoxid ≥ 0,9% | Strontiumoxid ≥ 0,1% |
| Vanadiumoxid ≥ 0,1% | Aluminiumoxid ≥ 0,1% |
| Magnesiumoxid ≥ 0,1% | Siliciumoxid ≥ 0,1% |
| Germaniumoxid ≥ 0,8% | Lanthanoxid ≥ 0,1% |
| $CaCO_3$ ≥ 0,1% | |

e)

| | |
|---|---|
| Chromoxid ≥ 94% | Eisen(III)-oxid ≥ 0,5% |
| Chrom ≥ 0,8% | Zinkoxid ≥ 0,5% |
| Kupferoxid ≥ 0,2% | Cobaltoxid ≥ 0,2% |
| Manganoxid ≥ 0,3% | Molybdänoxid ≥ 0,1% |
| Selenoxid ≥ 0,9% | Strontiumoxid ≥ 0,1% |
| Vanadiumoxid ≥ 0,1% | Aluminiumoxid ≥ 0,1% |
| Magnesium ≥ 0,1% | Siliciumoxid ≥ 0,1% |
| Germaniumoxid ≥ 0,8% | Lanthanoxid ≥ 0,1% |
| KI ≥ 0,1% | $BO_2$ ≥ 0,1% |
| $CaCO_3$ ≥ 0,1% | $MgF_2$ ≥ 0,1% |

f)

| | |
|---|---|
| Chromoxid ≥ 94% | Eisen(III)-oxid ≥ 0,5% |
| Chrom ≥ 0,8% | Zinkoxid ≥ 0,5% |
| Kupferoxid ≥ 0,1% | Cobaltoxid ≥ 0,1% |
| Manganoxid ≥ 0,1% | Molybdänoxid ≥ 0,1% |
| Selenoxid ≥ 0,9% | Strontiumoxid ≥ 0,1% |
| Vanadiumoxid ≥ 0,1% | Aluminiumoxid ≥ 0,1% |
| Magnesiumoxid ≥ 0,1% | Siliciumoxid ≥ 0,1% |
| Germaniumoxid ≥ 0,8% | Lanthanoxid ≥ 0,1% |

und eine kleine Menge von Lithium, Kalium oder Natrium, die nicht mehr als 0,5% beträgt.

g)

| | |
|---|---|
| Chromoxid ≥ 92% | Eisen(III)-oxid ≥ 0,5% |
| Chrom ≥ 0,8% | Zinkoxid ≥ 0,5% |
| Kupferoxid ≥ 0,1% | Cobaltoxid ≥ 0,1% |
| Manganoxid ≥ 0,2% | Molbdänoxid ≥ 1% |
| Selenoxid ≥ 1% | Strontiumoxid ≥ 0,1% |
| Vanadiumoxid ≥ 0,1% | Lanthan ≥ 0,1% |
| KI ≥ 0,1% | |

und eine geeignete Menge von Titan oder Bor, jeweils in einer Menge, die nicht mehr als 0,2% beträgt.

h)

| | |
|---|---|
| Chromoxid ≥ 90% | Eisen(III)-oxid ≥ 1% |
| Chrom ≥ 1% | Zinkoxid ≥ 1% |
| Kupferoxid ≥ 0,2% | Cobaltoxid ≥ 0,2% |
| Manganoxid ≥ 0,5% | Molybdänoxid ≥ 0,1% |
| Selenoxid ≥ 1,5% | Strontiumoxid ≥ 0,1% |
| Vanadiumoxid ≥ 0,1% | Aluminiumoxid ≥ 0,2% |
| Magnesiumoxid ≥ 0,8% | Siliciumoxid ≥ 0,3% |
| Germaniumoxid ≥ 1% | Lanthan ≥ 0,1% |
| KI ≥ 0,1% | |

i)

| | |
|---|---|
| Selenoxid ≥ 5% | Germaniumoxid ≥ 5% |
| Siliciumoxid ≥ 50% | Thoriumoxid ≥ 4% |
| Ceroxid ≥ 5% | Zirconiumoxid ≥ 15% |
| Bornitrid ≥ 10% | Titancarbid ≥ 3% |
| Wolframcarbid ≥ 3% | Platin ≥ 0,01% |

j)

Aluminiumoxid ≥ 85%        Eisen(III)-oxid ≥ 3%
Cobaltoxid ≥ 2%            Titanoxid ≥ 3%
Siliciumoxid ≥ 3%          Hafniumoxid ≥ 2%
Platin ≥ 0,01%

k)

Yttriumoxid ≥ 5%           ReCaMnFeOx ≥ 88%
Siliciumoxid ≥ 5%

l)

Chromoxid ≥ 95%            Chrom ≥ 1%
Zinkoxid ≥ 1%             Siliciumoxid ≥ 1%
Ceroxid ≥ 1%

m)

Titanoxid ≥ 90%            Zirconiumoxid ≥ 5%
Siliciumoxid ≥ 1%          Eisen(III)-oxid ≥ 1,5%
Zinkoxid ≥ 1%             Kupferoxid ≥ 1%
Berylliumoxid ≥ 0,5%

n)

Eisen(III)-oxid ≥ 20%       Zirconiumoxid ≥ 75%
Siliciumoxid ≥ 2%          Zinoxid ≥ 1%
Niobiumoxid ≥ 2%

o)

Siliciumoxid ≥ 80%         Eisen(III)-oxid ≥ 2%
Vanadiumoxid ≥ 2%         Zinkoxid ≥ 2%
Titanoxid ≥ 2%            Bornitrid ≥ 2%
Wolframcarbid ≥ 1%        Magnesiumoxid ≥ 2%
Wolframoxid ≥ 5%          Ceroxid ≥ 2%

p)

Aluminiumoxid ≥ 80%       Ceroxid ≥ 1%
Eisen(III)-oxid ≥ 5%        Cobaltoxid ≥ 3%
Chromoxid ≥ 5%           Siliumoxid ≥ 1%

2. Vorrichtung nach Anspruch 1, worin das strahlungserzeugende Mittel ein nichtmetallisches Substrat; eine Schicht, die aus der Zusammensetzung zusammengesetzt ist und auf die Oberfläche des Substrats aufgebracht ist, und ein elektrothermisches Umwandlungsmittel, das in dem Substrat bereitgestellt ist, umfaßt.

3. Vorrichtung nach Anspruch 1, worin das strahlungserzeugende Mittel ein nichtmetallisches Substrat; eine leitende Membran aus Metalloxiden, die auf die Oberfläche des Substrats aufgebracht sind, um ein elektrothermisches Energieumwandlungsmittel zu bilden; und eine Schicht, die aus der Zusammensetzung zusammengesetzt ist, welche auf die Oberfläche der leitenden Membran beschichtungsmäßig aufgetragen ist, umfaßt.

4. Vorrichtung nach Anspruch 1, worin das strahlungserzeugende Mittel ein nichtmetallisches Substrat, enthaltend darin die Zusammensetzung, und ein elektrothermisches Energieumwandlungsmittel umfaßt.

5. Vorrichtung nach Anspruch 1, worin das strahlungserseugende Mittel ein nichtmetallisches Substrat, enthaltend darin die Zusammensetzung, und elektrisch leitende Substanzen zum Umwandeln von elektrischer Energie in thermische Energie umfaßt.

6.  Vorrichtung nach Anspruch 1, worin das strahlungserseugende Mittel ein Metallsubstrat, eine Schicht, die aus der Zusammensetzung zusammengesetzt ist und direkt auf die Oberfläche des Substrats aufgebracht ist oder in Form einer Emailmasse darauf beschichtungsmäßig aufgetragen ist, und ein elektrothermisches Energieumwandlungsmittel, welches in dem Substrat bereitgestellt ist, umfaßt.

7.  Vorrichtung nach Anspruch 1, worin das strahlungserzeugende Mittel ein Substrat, das aus hochtemperaturbeständigem Glas hergestellt ist, welches darin die Zusammensetzung enthält, und eine Schicht einer elektrisch leitenden Membran, die aus Metalloxiden hergestellt ist und auf der Oberfläche des Substrats gebildet ist, zum Umwandeln von elektrischer Energie in thermische Energie umfaßt.

8.  Vorrichtung nach Anspruch 1, worin das energieerzeugende Mittel eine Quelle eines Magnetfelds ist; und worin das strahlungserzeugende Mittel ein Substrät, das aus magnetischem Material hergestellt ist, und einen Überzug, der aus der Zusammensetzung zusammengesetzt und auf das Substrat aufgetragen ist, umfaßt.

**Revendications**

1.  Appareil pour la régulation et l'amélioration de l'état de croissance et de survie d'organismes vivants, ledit appareil comprenant :

    a) une source d'énergie (1);
    b) un moyen transducteur d'énergie (2) qui convertit l'énergie fournie par ladite source d'énergie (1) en une énergie thermique ou magnétique; et
    c) un moyen générateur de rayonnement (4) excité par ladite énergie thermique ou magnétique fournie par ledit moyen transducteur d'énergie (2) pour générer un rayonnement électromagnétique appelé biospectre simulé;
    caractérisé en ce que ledit moyen générateur de rayonnement (4) est constitué par une composition comprenant plusieurs éléments ou composés chimiques mélangés selon une proportion de mélange prédéterminée, ladite composition et ladite proportion de mélange relative étant choisies parmi les compositions et proportions de mélange suivantes :

    (a)

    | | |
    |---|---|
    | oxyde de chrome $\geq$ 95% | oxyde de sélénium $\geq$ 1% |
    | chrome $\geq$ 0,8% | oxyde de germanium $\geq$ 0,8% |
    | oxyde de zinc $\geq$ 0,5% | oxyde ferrique $\geq$ 0,5% |
    | oxyde de magnésium $\geq$ 0,2% | oxyde de cuivre $\geq$ 0,1% |
    | oxyde de cobalt $\geq$ 0,1% | oxyde de manganèse $\geq$ 0,1% |
    | oxyde de molybdène $\geq$ 0,1% | oxyde de strontium $\geq$ 0,1% |
    | oxyde de vanadium $\geq$ 0,1% | oxyde d'aluminium $\geq$ 0,1% |
    | oxyde de silicium $\geq$ 0,1% | lanthane $\geq$ 0,1% |
    | fluorure de magnésium $\geq$ 0,1% | |

    (b)

    | | |
    |---|---|
    | oxyde de chrome $\geq$ 95% | oxyde ferrique $\geq$ 0,5% |
    | chrome $\geq$ 0,8% | oxyde de zinc $\geq$ 0,4% |
    | oxyde de cuivre $\geq$ 0,1% | oxyde de cobalt $\geq$ 0,1% |
    | oxyde de manganèse $\geq$ 0,1% | oxyde de molybdène $\geq$ 0,1% |
    | oxyde de sélénium $\geq$ 0,7% | oxyde de strontium $\geq$ 0,1% |
    | oxyde de vanadium $\geq$ 0,1% | oxyde d'aluminium $\geq$ 0,1% |
    | oxyde de magnésium $\geq$ 0,1% | oxyde de silicium $\geq$ 0,1% |
    | oxyde de chrome $\geq$ 0,8% | lanthane $\geq$ 0,1% |
    | $BO_2 \geq$ 0,1% | $MgF_2 \geq$ 0,1% |

    (c)

    | | |
    |---|---|
    | oxyde de chrome $\geq$ 93% | oxyde ferrique $\geq$ 0,5% |
    | chrome $\geq$ 0,8% | oxyde de zinc $\geq$ 0,5% |

| | |
|---|---|
| oxyde de cuivre $\geq$ 0,1% | oxyde de cobalt $\geq$ 0,1% |
| oxyde de manganèse $\geq$ 0,1% | oxyde de molybdène $\geq$ 0,1% |
| oxyde de sélénium $\geq$ 1% | oxyde de strontium $\geq$ 0,1% |
| oxyde de vanadium $\geq$ 0,1% | oxyde d'aluminium $\geq$ 0,1% |
| oxyde de magnésium $\geq$ 0,1% | oxyde de silicium $\geq$ 0,1% |
| oxyde de germanium $\geq$ 1% | oxyde de lanthane $\geq$ 0,1% |
| $MgF_2 \geq$ 0,1% | |

et des quantités appropriées de lithium, de potassium, ou de titane non supérieures à 0,2% pour chaque élément.

(d)

| | |
|---|---|
| oxyde de chrome $\geq$ 94% | oxyde ferrique $\geq$ 0,5% |
| chrome $\geq$ 0,8% | oxyde de zinc $\geq$ 0,7% |
| oxyde de cuivre $\geq$ 0,2% | oxyde de cobalt $\geq$ 0,2% |
| oxyde de manganèse $\geq$ 0,3% | oxyde de molybdène $\geq$ 0,1% |
| oxyde de sélénium $\geq$ 0,9% | oxyde de strontium $\geq$ 0,1% |
| oxyde de vanadium $\geq$ 0,1% | oxyde d'aluminium $\geq$ 0,1% |
| oxyde de magnésium $\geq$ 0,1% | oxyde de silicium $\geq$ 0,1% |
| oxyde de germanium $\geq$ 0,8% | oxyde de lanthane $\geq$ 0,1% |
| $CaCO_3 \geq$ 0,1% | |

(e)

| | |
|---|---|
| oxyde de chrome $\geq$ 94% | oxyde ferrique $\geq$ 0,5% |
| chrome $\geq$ 0,8% | oxyde de zinc $\geq$ 0,5% |
| oxyde de cuivre $\geq$ 0,2% | oxyde de cobalt $\geq$ 0,2% |
| oxyde de manganèse $\geq$ 0,3% | oxyde de molybdène $\geq$ 0,1% |
| oxyde de sélénium $\geq$ 0,9% | oxyde de strontium $\geq$ 0,1% |
| oxyde de vanadium $\geq$ 0,1% | oxyde d'aluminium $\geq$ 0,1% |
| magnésium $\geq$ 0,1% | oxyde de silicium $\geq$ 0,1% |
| oxyde de germanium $\geq$ 0,8% | oxyde de lanthane $\geq$ 0,1% |
| $KI \geq$ 0,1% | $BO_2 \geq$ 0,1% |
| $CaCO_3 \geq$ 0,1% | $MgF_2 \geq$ 0,1% |

(f)

| | |
|---|---|
| oxyde de chrome $\geq$ 94% | oxyde ferrique $\geq$ 0,5% |
| chrome $\geq$ 0,8% | oxyde de zinc $\geq$ 0,5% |
| oxyde de cuivre $\geq$ 0,1% | oxyde de cobalt $\geq$ 0,1% |
| oxyde de manganèse $\geq$ 0,1% | oxyde de molybdène $\geq$ 0,1% |
| oxyde de sélénium $\geq$ 0,9% | oxyde de strontium $\geq$ 0,1% |
| oxyde de vanadium $\geq$ 0,1% | oxyde d'aluminium $\geq$ 0,1% |
| oxyde de magnésium $\geq$ 0,1% | oxyde de silicium $\geq$ 0,1% |
| oxyde de germanium $\geq$ 0,8% | oxyde de lanthane $\geq$ 0,1% |

et une faible quantité de lithium, de potassium, ou de sodium non supérieure à 0,5%.

(g)

| | |
|---|---|
| oxyde de chrome $\geq$ 92% | oxyde ferrique $\geq$ 0,5% |
| chrome $\geq$ 0,8% | oxyde de zinc $\geq$ 0,5% |
| oxyde de cuivre $\geq$ 0,1% | oxyde de cobalt $\geq$ 0,1% |
| oxyde de manganèse $\geq$ 0,2% | oxyde de molybdène $\geq$ 1% |
| oxyde de sélénium $\geq$ 1% | oxyde de strontium $\geq$ 0,1% |
| oxyde de vanadium $\geq$ 0,1% | lanthane $\geq$ 0,1% |
| $KI \geq$ 0,1% | |

et une quantité appropriée de titane ou de bore respectivement non supérieure à 0,2%.

(h)

| | |
|---|---|
| oxyde de chrome ≥ 90% | oxyde ferrique ≥ 1% |
| chrome ≥ 1% | oxyde de zinc ≥ 1% |
| oxyde de cuivre ≥ 0,2% | oxyde de cobalt ≥ 0,2% |
| oxyde de manganèse ≥ 0,5% | oxyde de molybdène ≥ 0,1% |
| oxyde de sélénium ≥ 1,5% | oxyde de strontium ≥ 0,1% |
| oxyde de vanadium ≥ 0,1% | oxyde d'aluminium ≥ 0,2% |
| oxyde de magnésium ≥ 0,8% | oxyde de silicium ≥ 0,3% |
| oxyde de germanium ≥ 1% | lanthane ≥ 0,1% |
| KI ≥ 0,1% | |

(i)

| | |
|---|---|
| oxyde de sélénium ≥ 5% | oxyde de germanium ≥ 5% |
| oxyde de silicium ≥ 50% | oxyde de thorium ≥ 4% |
| oxyde de cérium ≥ 5% | oxyde de zirconium ≥ 15% |
| nitrure de bore ≥ 10% | carbure de titane ≥ 3% |
| carbure de tungstène ≥ 3% | platine ≥ 0,01% |

(j)

| | |
|---|---|
| oxyde d'aluminium ≥ 85% | oxyde ferrique ≥ 3% |
| oxyde de cobalt ≥ 2% | oxyde de titane ≥ 3% |
| oxyde de silicium ≥ 3% | oxyde d'hafnium ≥ 2% |
| platine ≥ 0,01% | |

(k)

| | |
|---|---|
| oxyde d'yttrium ≥ 5% | ReCaMnFeOx ≥ 88% |
| oxyde de silicium ≥ 5% | |

(l)

| | |
|---|---|
| oxyde de chrome ≥ 95% | chrome ≥ 1% |
| oxyde de zinc ≥ 1% | oxyde de silicium ≥ 1% |
| oxyde de cérium ≥ 1% | |

(m)

| | |
|---|---|
| oxyde de titane ≥ 90% | oxyde de zirconium ≥ 5% |
| oxyde de silicium ≥ 1% | oxyde ferrique ≥ 1,5% |
| oxyde de zinc ≥ 1% | oxyde de cuivre ≥ 1% |
| oxyde de béryllium ≥ 0,5% | |

(n)

| | |
|---|---|
| oxyde ferrique ≥ 20% | oxyde de zirconium ≥ 75% |
| oxyde de silicium ≥ 2% | oxyde de zinc ≥ 1% |
| oxyde de niobium ≥ 2% | |

(o)

| | |
|---|---|
| oxyde de silicium ≥ 80% | oxyde ferrique ≥ 2% |
| oxyde de vanadium ≥ 2% | oxyde de zinc ≥ 2% |
| oxyde de titane ≥ 2% | nitrure de bore ≥ 2% |

| carbure de tungstène ≥ 1% | oxyde de magnésium ≥ 2% |
| oxyde de tungstène ≥ 5% | oxyde de cérium ≥ 2% |

(p)

| oxyde d'aluminium ≥ 80% | oxyde de cérium ≥ 1% |
| oxyde ferrique ≥ 5% | oxyde de cobalt ≥ 3% |
| oxyde de chrome ≥ 5% | oxyde de silicium ≥ 1% |

2. Appareil selon la revendication 1, dans lequel ledit moyen générateur de rayonnement comprend un substrat non métallique; une couche composée de ladite composition et plaquée sur la surface dudit substrat; et un moyen de conversion électrothermique prévu à l'intérieur dudit substrat.

3. Appareil selon la revendication 1, dans lequel ledit moyen générateur de rayonnement comprend un substrat non métallique; une membrane conductrice en oxydes métalliques plaquée sur la surface dudit substrat pour former un moyen convertisseur d'énergie électrothermique; et une couche composée de ladite composition appliquée en revêtement sur la surface de ladite membrane conductrice.

4. Appareil selon la revendication 1, dans lequel ledit moyen générateur de rayonnement comprend un substrat non métallique contenant ladite composition et un moyen convertisseur d'énergie électrothermique.

5. Appareil selon la revendication 1, dans lequel ledit moyen générateur de rayonnement comprend un substrat non métallique contenant ladite composition, et des substances électriquement conductrices pour convertir une énergie électrique en une énergie thermique.

6. Appareil selon la revendication 1, dans lequel ledit moyen générateur de rayonnement comprend un substrat métallique, une couche composée de ladite composition et plaquée directement sur la surface du substrat ou appliquée sur celui-ci en revêtement sous la forme d'une pulpe d'émail et un moyen convertisseur d'énergie électrothermique prévu à l'intérieur dudit substrat.

7. Appareil selon la revendication 1, dans lequel ledit moyen générateur de rayonnement comprend un substrat réalisé à partir d'un verre résistant aux températures élevées, contenant ladite composition, et une couche formée d'une membrane électriquement conductrice réalisée à partir d'oxydes métalliques et formée sur la surface du substrat pour convertir une énergie électrique en une énergie thermique.

8. Appareil selon la revendication 1, dans lequel ledit moyen générateur d'énergie est une source de champ magnétique; et dans lequel ledit moyen générateur de rayonnement comprend un substrat réalisé à partir d'une matière magnétique et un revêtement composé de ladite composition et appliqué en peinture sur ledit substrat.

Fig.1

A. Transition of electron
B. Rotation of molecules
C. Vibration of atoms

Simulated bio-spectrum
Bio-spectrum

Fig.2

0.72-3 µm Nearinfrared
0.72-50 µm Near-far infrared
0.45-10⁵ µm Simulated bio-spectrum

Fig.3

1. Energy source
2. Simulated bio-spectrum generator
3. Energy transducer
4. Simulated bio-spectrum generating component

Fig. 4A-1

Fig.4A-2

Fig.4B-1

Fig.4B-2

Fig.4C-1

Fig.4C-2

Fig.4D

Fig.4

Fig.5A

Fig.5B

Fig.5C

Fig.5